# EUROPÄISCHE PATENTSCHRIFT

(11) **EP 4 028 069 B1**
(45) Veröffentlichungstag und Bekanntmachung des Hinweises auf die Patenterteilung: **20.05.2026**
(21) Anmeldenummer: 20706809.9
(22) Anmeldetag: 16.01.2020
(51) Int. Cl.: A61M 1/00, A61M 3/02, A61F 5/441

(54) **VORRICHTUNG ZUR KONTINUIERLICHEN ABLEITUNG VON GAS DURCH EINE NATÜRLICHE ÖFFNUNG DES KÖRPERS EINES PATIENTEN**
APPARATUS FOR CONTINUOUSLY REMOVING GAS THROUGH A NATURAL OPENING IN THE BODY OF A PATIENT
DISPOSITIF D'ÉVACUATION CONTINUE DE GAZ PAR UNE OUVERTURE NATURELLE DU CORPS D'UN PATIENT

(30) Priorität: 10.09.2019 DE 102019006377
(43) Veröffentlichungstag der Anmeldung: 20.07.2022
(73) Patentinhaber: Advanced Medical Balloons GmbH, 68753 Waghäusel (DE)
(72) Erfinder: GÖBEL, Fred, 67346 Speyer (DE)
(74) Vertreter: Schott, Jakob Valentin
(86) Internationale Anmeldenummer: PCT/IB2020/050337
(87) Internationale Veröffentlichungsnummer: WO 2021/048635

(56) Entgegenhaltungen:
- WO-A1-2017/066092
- DE-U1- 20 107 624
- US-A1- 2005 143 696
- US-A1- 2014 323 990

## Beschreibung

Die vorliegende Erfindung beschreibt ein System zur geschlossenen Ableitung und/oder Sammlung von Stuhl oder sonstigen, stark geruchsbildenden Exkreten oder Sekreten und zur möglichst geruchsneutralen Ableitung von Darmgas oder sonstigen, übelriechende Gasen durch eine natürliche Öffnung des Körpers eines menschlichen oder tierischen Patienten, mit einem ableitenden, schlauch- oder rohrförmigen Drainagekatheter, einer stromabwärts des Drainagekatheters positionierten Vorrichtung zur möglichst geruchsneutralen Ableitung des Darmgases oder der sonstigen, übelriechenden Gase, und einem stromabwärts der Vorrichtung und daran extrakorporal angeschlossenen Sammelbeutel, um den Stuhl oder die abgeleiteten Exkrete oder Sekrete in ein Sammelkompartiment innerhalb des Sammelbeutels abzuleiten.

Systeme zur kontinuierlichen Ableitung von Stuhl aus dem Rektum eines Patienten sind ein etablierter Bestandteil der modernen medizinischen bzw. pflegerischen Versorgung. Die heute bekannten stuhlableitenden Systeme weisen im typischen Aufbau eine torus- oder ballonartig gestaltete Kopfeinheit auf, die die jeweilige Vorrichtung im Rektum des Patienten verankert (retiniert). Die Kopfeinheit verfügt ferner über eine, den Stuhl im Rektum aufnehmende und durch den Anus bzw. den analen Schließmuskel hindurchleitende Schlauchkomponente, auf dem das die Drainage im Rektum retinierende Torus- oder Ballonelement distal endständig fixiert ist. Der rektal ankernden und den Stuhl trans-anal ableitenden Kopfeinheit schließt sich ein weiteres, extrakorporales Schlauchsegment an, das den Stuhl vom Patienten in ein, in der Regel beutelförmiges Sammelgefäß weiterleitet, welches typischerweise auf einem Niveau unterhalb des Patienten am Bettrahmen angebracht wird, wodurch der Stuhl einem Gefälle folgend abläuft. Der Beutel ist in aller Regel wechselbar und verfügt über ein entsprechendes, manuell konnektierendes Verschluss-System zur Anbindung an die Drainageeinheit. Die Konnektion kann beispielsweise als bajonettartiger Verschluss ausgeführt sein. Der stuhlsammelnde Beutel weist in der Regel eine entgasende Funktion bzw. Komponente auf, die im Darm des Patienten entstehendes Darmgas aus dem Drainagesystem in die Umgebung ablässt bzw. eine Stauung von Darmgas im Abdomen vermeidet, indem sie das drainierende System fortlaufend zur Umgebung hin dekomprimiert. Die suffiziente Dekompression von Darmgas hat im Anwendungsverlauf eine hohe klinische Relevanz, da sich Darmgase u.U. bis in den hohen Dünndarm aufstauen und eine Ileus-artige Symptomatik auslösen können. Stauungsbedingter Meteorismus kann ebenfalls zu Beeinträchtigungen der Zwerchfellbeweglichkeit führen und so die maschinelle Beatmung des Patienten beeinträchtigen. Die entgasende Funktion bzw. Komponente des Beutels ist häufig mit einem Adsorber oder Filter ausgestattet, der fäkal, fötide oder sonstig unangenehm riechende Geruchsstoffe bindet.

Stuhldrainagen des herkömmlichen Bautyps sind häufig bei großen abzuleitenden Gasmengen oder bei besonders intensiv riechenden Stühlen nicht in der Lage, eine suffiziente Dekompression oder Geruchsbindung (Adsorption) zu gewährleisten. Dies ist in der Regel durch eine unzureichende Menge adsorbierenden bzw. filternden Materials bedingt, aber auch durch die typische Positionierung der gasablassenden Komponente innerhalb des stuhlsammelnden Raumes des Sammelbeutels verursacht. Die entgasende Funktion eines in der Beutelwandung integrierten Filters kann durch eine passagere, flache Lagerung des Beutels beeinträchtigt werden, wie diese beispielsweise beim Transport oder bei der Lagerung des Patienten häufig vorkommt. In einer liegenden Position des Beutels gelangt Beutelinhalt an die gasauslassende Komponente und verschmutzt bzw. benetzt deren Innenseite, was die Fähigkeit zur Dekompression des Gesamtsystems einschränken kann. Das jeweils verwendete Filter- oder Adsorbermaterial kann sich zudem mit Flüssigkeit vollsaugen und so dauerhaft seine gasablassende, dekomprierende Funktion verlieren.

In der US 2014 / 0 323 990 A1 wird ein System zum Handhaben von Exkreten oder Sekreten aus dem menschlichen Körper beschrieben, mit einem ableitenden Katheter, einer Drainageleitung und einem Sammelbehälter. Allerdings ist hier ein Geruchsfilter mit dem Sammelbehälter integriert und muss zusammen mit jenem entsorgt werden. Darüber hinaus fehlt es an automatischen Verschlussvorrichtung an der Öffnung des Sammelbehälters, welche beim Wechseln des Sammelbehälters das Entweichen von Gasen oder von Flüssigkeit oder sonstigem Material verhindern könnte.

Die US 2005 / 0 143 696 A1 offenbart eine Stoma-Vorrichtung, bestehend aus aus zwei flächigen Elementen randseitig zusammengeschweissten Stoma-Beutel mit einer seitlichen, dem Anus präter zugewandten Öffnung, die von einer platten- oder pflasterartigen, klebenden Struktur umschlossen wird, ansonsten aber vollkommen offen ist, und mit einem Geruchsfilter im Bereich der Öffnung. Hier fehlt es an einem ableitenden Katheter, einer schlauchförmigen Drainageleitung, und beim Lösen des Stoma-Beutels von der klebenden Struktur fehlt es an einer automatisierten Verschlussvorrichtung, welche das Entweichen von Gasen oder von Flüssigkeit oder sonstigem Material verhindern könnte. Der Filter ist nicht exponiert, sondern mit der klebenden Struktur oder dem Stomabeutel integriert und muss zusammen mit jenem entsorgt werden.

Die WO 2017 / 066 092 A1 beschreibt ebenfalls einen Stomabeutel, der von einem Patienten flach an der Hautoberfläche getragen werden kann. Hier fehlt ein ableitender Katheter völlig.

Die DE 201 07 624 U1 offenbart einen Geruchsfilter für Stomabeutel mit einem Absorptionsmaterial, einem Gaseinlass und einem Gasauslass, welch emit einer Membran verschlossen sind, wobei der filter flach ausgebildet ist und das Absorptionsmaterial in einen von dem Filter ausgebildeten spiralförmigen Gang eingebracht ist.. Dieser Geruchsfilter wird im oder am Stomabeutel angebracht.

Demzufolge muss bei jedem Wechsel des Stomabeutels stets der Filter ausgewechselt werden, der somit als billiges Wegwerfprodukt gestaltet sein muss.

Über die sonstigen Gestaltungsmerkmale des Stomabeutels - insbesondere ob jener mit einem speziellen Verschluss ausgebildet ist od. dgl. - findet sich bei diesem Dokument keinerlei Hinweis.

Aus den Nachteilen des geschilderten Standes der Technik resultiert das die Erfindung initiierende Problem, im Rahmen eines gattungsgemäßen Systems zur Drainage und/oder Sammlung von Stuhl oder sonstigen, stark geruchsbildenden Exkreten oder Sekreten aus dem menschlichen oder tierischen Körper dafür Sorge zu tragen, dass von diesem System möglichst keine Geruchsbeeinträchtigung ausgeht.

Die Lösung dieses Problems gelingt bei einer gattungsgemäßen Vorrichtung durch eine Weiterbildung gemäß dem Kennzeichen des beigefügten Hauptanspruchs.

Die Erfindung zeichnet sich dadurch aus, dass der Sammelbeutel über eine rückflusshemmende Funktion verfügt, die bereits in den Sammelbeutel gelangtes Material dort hält, auch wenn der Sammelbeutel aus der vertikalen Normallage in eine flach liegende Position gelangt, wobei die Vorrichtung eine schlauch- oder rohrförmige Struktur nach Art eines T-Stücks, welche neben (i) einem distalen, zum Patienten gerichteten Ende als Zulaufanschluss, einem proximalen, dem Zulaufanschluss in axialer Verlängerung gegenüber stehenden, zum Sammelbeutel gerichteten Ende als Ablaufanschluss und (iii) einem jene verbindenden Durchleitungsbereich für die Durchleitung von Stuhl oder sonstigen Exkreten oder Sekreten eine lateral zur Umgebung gerichtete Abzweigung aufweist, wo Darmgas oder sonstige, übelriechende Gase von dem Stuhl oder den sonstigen Exkreten oder Sekreten getrennt und durch eine Adsorptions- und/oder Absorptions- und/oder Filtereinrichtung zu einem Auslass geleitet werden.

Somit beschreibt die Erfindung eine Vorrichtung zur Adsorption und/oder Filterung von im Körper gebildeten, stark riechenden Gasen, welche in einem System zur kontinuierlichen, geschlossenen Ableitung und Sammlung von Stuhl, oder auch für die Ableitung anderer, stark geruchsbildender Exkrete oder Sekrete, integriert ist. Die Vorrichtung ist für eine kontinuierlich effiziente, positionierungsunabhängige Entgasungsfunktion ausgelegt. Die abgeleiteten Exkrete oder Sekrete werden dabei innerhalb der Vorrichtung derart durch das rohr- oder schlauchartige sowie T-Stückartig ausgebildete Grundelement der Vorrichtung geführt, dass bei annähernd vertikaler Normallage der Vorrichtung eine Verschmutzung oder ein Verschluss der das Gas zur Umgebung hin ablassenden Komponenten weitgehend vermieden, und Stauungen von beispielsweise Darmgas innerhalb des Systems verhindert werden können. Die Vorrichtung kann optional fest in das System integriert sein, oder auch als gasdicht konnektierbare, akzessorische Einheit bei Bedarf vom Anwender in das System eingebaut werden. Die Vorrichtung verfügt bevorzugt über eine Trennlage zum adsorbierenden und/oder filternden gerichtete Medium, die Flüssigkeit und/oder Stuhl abweist, jedoch für Gas durchlässig ist. Das adsorbierende und/oder filternde Medium ist optional in fixer oder lösbar, wechselbarer Bauweise in die Vorrichtung integriert. Die Vorrichtung ist zwischen dem ableitenden Katheter und der sammelnden Beuteleinheit positioniert, dem Beutelinhalt also nicht kontinuierlich exponiert. Der sammelnde Beutel verfügt über eine rückflusshemmende Funktion, die bereits in den Beutel gelangtes Material dort hält, auch wenn der Beutel aus der vertikalen Normallage in eine flach liegende Position gelangt. Die Vorrichtung gewährleistet ferner, dass in den sammelnden Raum des Gefäßes vorgedrungenes Gas, eine im Gefäß integrierte, rückflusshemmende Funktion überwindet, und der dem sammelnden Gefäß vorgeschalteten, gasablassenden Vorrichtung zugeführt wird.

Die im Rahmen der Erfindung beschriebene Vorrichtung zum Ablassen von im Körper gebildeten Gasmengen weist ein Grundelement nach Art eines T-Stücks auf, wobei das obere, distale, zum Patienten gerichtete Ende dem unteren, proximalen, zum Sammelbeutel gerichteten Ende des T-Stücks in axialer Verlängerung direkt gegenübersteht. Der freie, nach lateral zur Umgebung gerichtete und geöffnete Schenkel des T-Stücks nimmt die geruchsadsorbierende und/oder filternde, mit einer entsprechenden Substanz, beispielsweise auf Aktivkohle-Basis, befüllte Einheit auf. Neben oberflächenaktiven, die Geruchsstoffe auf der Oberfläche anlagernden, adsorbierenden Substanzen, kommen ebenfalls absorbierende, die geruchswirksamen Stoffe in Art einer physikalischen "Lösung" aufnehmende Substanzen in Frage. Die Einheit kann in fixer Bauweise mit dem lateralen Schenkel verbunden sein, oder durch eine entsprechende Konnektion an diesen konnektiert, beispielsweise aufgesteckt sein.

Es ist für die Funktion der Erfindung wesentlich, dass bei normaler, vertikaler Positionierung der entgasenden Baugruppe im stuhlableitenden System Darmgas ungehindert und auch bei großen Gasmengen effizient aus dem System austreten kann. Kommt es zu einem Wechsel aus der vertikalen Normallage der Baugruppe in eine passagere Horizontallage, soll ein eventueller Verschluss der für die Entgasung erforderlichen Komponenten durch Stuhl so wirksam wie möglich vermieden werden. Das adsorbierende und/oder filternde Material wird im Rahmen der Erfindung durch verschiedene bauliche Merkmale in vom in der Schlauchleitung abgeleiteten und im Beutel gesammelten Stuhl getrennt. Die Erfindung beschreibt eine grundsätzliche Bauform der Vorrichtung, bei der bereits in den Sammelbehälter bzw. Sammelbeutel geleiteter Inhalt bei einer Horizontallage am Rückstrom zur entgasenden Baugruppe, beispielsweise durch folienbasierte, rückschlaghemmende, lippen- oder segelartige Komponenten gehindert wird, und die Baugruppe zur Entgasung dem Sammelbehälter prinzipiell baulich vorgeschaltet wird.

Es liegt im Rahmen der Erfindung, dass die Längsrichtung des Strömungskanals zwischen der Abzweigung und der Adsorptions- und/oder Absorptions- und/oder Filtereinrichtung mit der Längsrichtung des Durchleitungsbereichs zwischen der Abzweigung und dem Ablaufanschluss einen Winkel von 90° oder weniger als 90° einschließt, beispielsweise einen Winkel von 75° oder weniger, vorzugsweise einen Winkel von 60° oder weniger, insbesondere einen Winkel von 45° oder weniger, oder sogar einen Winkel von 30° oder weniger. Falls der besagte Winkel kleiner als 90° ist, so ist die Längsrichtung des Strömungskanals zwischen der Abzweigung und der Adsorptions- und/oder Absorptions- und/oder Filtereinrichtung bevorzugt ansteigend geneigt, d.h., zu dem Zulaufanschluss hin versetzt ist.

Der maximale Strömungsquerschnitt in dem Bereich zwischen der Abzweigung und der Adsorptions- und/oder Absorptions- und/oder Filtereinrichtung kann gleich oder kleiner sein als der minimale Strömungsquerschnitt in dem Durchleitungsbereich. Eine derrtige Verjüngung ist dabei unproblematisch, weil durch die Adsorptions-und/oder Absorptions- und/oder Filtereinrichtung ohnehin nur Gase strömen sollen.

Diesem Zweck folgend, wonach der Bereich zwischen der Abzweigung und der Adsorptions- und/oder Absorptions- und/oder Filtereinrichtung nur als Strömungskanal für Gase dienen soll, kann erfindungsgemäß vorgesehen sein, dass der Strömungsquerschnitt in dem Bereich zwischen der Abzweigung und der Adsorptions- und/oder Absorptions- und/oder Filtereinrichtung gegenüber dem minimalen Strömungsquerschnitt in dem Durchleitungsbereich durch ein Hindernis verengt ist, welches zumindest festen Stoffen, vorzugsweise aber auch flüssigen Stoffen einen größeren Widerstand bietet als gasförmigen Stoffen.

Bevorzugt ist ein solches Hindernis als eine Zunge ausgebildet, die innerhalb des Durchleitungsbereichs etwa auf Höhe der Abzweigung angeordnet ist.

Die Erfindung lässt sich dahingehend weiterbilden, dass das Hindernis als eine Zunge ausgebildet ist, die stromaufwärts der Abzweigung befestigt ist und die Abzweigung zu der Adsorptions- und/oder Absorptions- und/oder Filtereinrichtung im Durchleitungsbereich im Falle der Durchleitung von Stuhl oder sonstigen Exkreten oder Sekreten zumindest bereichsweise abdeckt. Eine solche Zunge kann entweder nur an ihrer stromaufwärtigen Kante festgelegt sein oder auch an ihren beiden, daran grenzenden Kanten.

Die Zunge kann in begrenztem Umfang biegsam und/oder elastisch ausgebildet sein, und besteht vorzugsweise aus Kunststoff. Sie sollte entlang ihrer festgelegten Kanten derart verbunden sein, dass sie dadurch eine Vorzugsrichtung relativ zu der Längsrichtung des Durchleitungsbereichs erhält, die dank einer ihr innewohnenden ausreichend steifen Beschaffenheit auch unabhängig von der Orientierung des Durchleitungsbereichs stets eingehalten wird, damit die Abzweigung für Gase stets offengehalten ist. Der eigentliche Zweck der Zunge besteht darin, durchgeleitete Exkrete oder Sekrete von der Adsorptions- und/oder Absorptions- und/oder Filtereinrichtung fernzuhalten, gleichzeitig aber das Entweichen von Gasen zu der Adsorptions- und/oder Absorptions- und/oder Filtereinrichtung zu erlauben bzw. zu begünstigen. Für die Ableitung von Gas ist ein geringer Öffnungsquerschnitt im Bereich einer freien Kante der Zunge ausreichend. Ein solcher Öffnungsquerschnitt könnte auch dadurch sichergestellt sein, dass an der Rückseite der Zunge, die dem von dem Durchleitungslumen abzweigenden Lumen zugewandt ist, ein oder mehrere Noppen oder sonstige Erhebungen vorgesehen sind, welche einen Mindestabstand der Zunge von dem Rand der Ableitungsöffnung sicherstellt; natürlich könnten solche Noppen auch im Bereich des Randes der Ableitungsöffnung angeordnet sein und bei Annäherung der Zunge mit jener in Kontakt treten.

Die Zunge kann mit der schlauch- oder rohrförmige Struktur integriert oder durch Klebung oder eine andere Fügetechnik verbunden sein.

Bevorzugt ist der Übergangsbereich zwischen der Zunge und der schlauch- oder rohrförmige Struktur nicht gelenkig ausgebildet, sondern eher steif, damit die Zunge ihre Orientierung in einer vorgegebenen Richtung stets einhält.

Die Zunge kann ein oder mehrere Löcher zum Durchtritt von Gasen aufweisen.

Ferner kann die dem Durchleitungsbereich oder der Abzweigung zugewandte Einlassöffnung der Adsorptions- und/oder Absorptions- und/oder Filtereinrichtung durch eine gasdurchlässige, vorzugsweise aber wasserabweisende Trennlage abgedeckt sein. Hierbei handelt es sich um eine weiter eMaßnahme, um den Filter für Gase stets offen zu halten.

Eine bevorzugte Weiterbildung der Erfindung besteht darin, dass die Trennlage eine gering benetzbare Oberfläche auweist (Lotoseffekt), insbesondere an ihrer dem Durchleitungsbereich oder der Abzweigung zugewandten Seite, so dass das unerwünschte Anhaften von Exkreten oder Sekreten vermieden wird.

Die Trennlage lässt sich bspw. als mikroporöse Membran ausbilden, beispielsweise aus expandiertem Polytetrafluorethylen, beispielsweise mit einem maximalen Porendurchmesser von weniger als 1 mm, vorzugsweise mit einem maximalen Porendurchmesser von 100 µm oder weniger, insbesondere mit einem maximalen Porendurchmesser von 10 µm oder weniger.

Andererseits lässt sich die Trennlage auch aus einem Papier herstellen, inbesondere aus einem Vliespapier.

Es liegt im Rahmen der Erfindung, dass die Adsorptions- und/oder Absorptions-und/oder Filtereinrichtung eine oder mehrere oberflächenaktive, die Geruchsstoffe auf der Oberfläche anlagernde, adsorbierenden Substanzen, aufweist, und/oder eine oder mehrere absorbierende, die geruchswirksamen Stoffe in Art einer physikalischer Lösung aufnehmende Substanzen.

Beispielsweise kann die Adsorptions- und/oder Absorptions- und/oder Filtereinrichtung ein adsorbierendes oder absorbierendes oder filterndes Granulat umassen, wie Aktivkohle, zum Beispiel in einer Menge von 100 ml oder weniger, bevorzugt in einer Menge von 50 ml oder weniger, insbesondere in einer Menge von 20 ml oder weniger, oder in einer Menge von 10 ml oder weniger, oder gar in einer Menge von 5 ml oder weniger.

Es wird empfohlen, dass die Adsorptions- und/oder Absorptions- und/oder Filtereinrichtung das Granulat, beispielsweise die Aktivkohle, in einer Menge von 2 ml oder mehr enthält, bevorzugt in einer Menge von 5 ml oder mehr, insbesondere in einer Menge von 10 ml oder weniger, oder in einer Menge von 20 ml oder mehr, oder gar in einer Menge von 30 ml oder mehr.

Um das zu reinigende Gas definiert durch die Adsorptions- und/oder Absorptions-und/oder Filtereinrichtung zu führen, sollte diese in ihrem Inneren ein oder mehrere lamellenartige Strukturen aufweisen, welche die Funktion von Wänden in einem Labyrinth übernehmen können.

Ein besonders guter Reinigungs- oder Filtereffekt ergibt sich, wenn das zu reinigende Gas auf einem möglichst langen Weg durch die Adsorptions- und/oder Absorptions-und/oder Filtereinrichtung geführt wird. Dies kann man insbesondere dadurch erreichen, dass mehrere lamellenartigen Strukturen gegeneinander versetzt sind und/oder nach Art von verschränkten Fingern ineinander greifen.

Eine andere Methode, innerhalb der Adsorptions- und/oder Absorptions- und/oder Filtereinrichtung einen möglichst langen Weg für ein zu reinigendes Gas zu schaffen, besteht darin, dass die Adsorptions- und/oder Absorptions- und/oder Filtereinrichtung in ihrem Inneren wenigstens eine schnecken-, spiral- und/oder wendelförmige Struktur aufweist, so dass das Gas gezwungen ist, entlang dieser schnecken-, spiralund/oder wendelförmigen Struktur zu strömen.

Die Erfindung lässt sich dahingehend weiterbilden, dass die Adsorptions- und/oder Absorptions- und/oder Filtereinrichtung ein infektiöse Erreger filterndes Material aufweist, insbesondere im Bereich ihrer stromabwärtigen bzw. auslassseitigen Öffnung. Durch eine solche Maßnahme kann die Übertragung von Krankheiten eingeschränkt werden

Wenn die Adsorptions- und/oder Absorptions- und/oder Filtereinrichtung in einer auswechselbaren Kartusche oder in einer abnehmbaren Kappe angeordnet ist, kann sie nach Erschöpfung des Adsorptions- und/oder Absorptions- und/oder Filtermaterials mit wenigen handgriffen gegen eine neue ausgetauscht werden.

Die auswechselbare Kartusche oder die abnehmbaren Kappe sollte im Bereich ihrer freien Stirnseite oder ihres Umfangs eine oder mehrere Auslassöffnungen aufweisen, wo das gereinigte bzw. gefilterte Gas in die Umgebung entweichen kann.

Der Ersatz einer verbrauchten Kartusche oder Kappe gestaltet sich besonders einfach, wenn die auswechselbare Kartusche oder die abnehmbaren Kappe über einen Bajonettverschluss oder über einen Schraubverschluss mit der schlauch- oder rohrförmige Struktur stromabwärts von deren Abzweigung verbindbar ist.

Indem die auswechselbare Kartusche oder die abnehmbaren Kappe über ihren Verschluss wahlweise auch mit dem Zulaufanschluss der schlauch- oder rohrförmige Struktur verbindbar ist, kann sie zum Entsorgen eines befüllten Sammelbeutels als geruchsichter Verschluss auf den Zulaufanschluss appliziert werden.

In Weiterverfolgung des Erfindungsgedankens kann vorgesehen sein, dass die schlauch- oder rohrförmige Struktur und/oder die auswechselbare Kartusche und/oder die abnehmbaren Kappe wenigstens ein Kompartiment zur Aufnahme eines Duftstoffs aufweist. Durch derartige Duftstoffe kann einerseits das Raumklima verbessert werden; besonders werden dadurch jedoch eventuell nicht vollständig herausgefilterte Gerüche überdeckt.

Das Kompartiment zur Aufnahme eines Duftstoffs kann nach außen offen sein. Damit der Duftstoff jedoch möglichst erst während des Gebrauchs der erfindungsgemäßen Vorrichtung freigesetzt wird, kann das Kompartiment zur Aufnahme eines Duftstoffs mit einer abnehmbaren Abdeckung, beispielsweise in Form einer Abziehfolie, verschlossen sein.

Die Erfindung lässt sich dahingehend weiterbilden, dass das Kompartiment zur Aufnahme eines Duftstoffs ringförmig ausgebildet ist und eine zentrale Auslassöffnung der Adsorptions- und/oder Absorptions- und/oder Filtereinrichtung ringförmig umgibt.

Im Rahmen einer derartigen Ausführungsform kann innerhalb der zentralen Öffnung des ringförmigen Kompartiments zur Aufnahme eines Duftstoffs ein Docht oder ein vliesartiges und/oder weitmaschiges und/oder großporiges Gewebe angeordnet ist, bspw. ein quer über die zentrale Öffnung gespannter Docht oder ein vliesartiges und/oder weitmaschiges und/oder großporiges Gewebe, der (das) sich zu seiner Befeuchtung mit Duftstoff bis in das den Duftstoff enthaltende Kompartiment hinein erstreckt.

Ferner kann die schlauch- oder rohrförmige Struktur zum Anschluss eines Schlauchs oder Sammelgefäßes oder -beutels od. dgl. an ihrem Zulaufanschluss und/oder an ihrem Ablaufanschluss einen Bajonettverschluss oder einen Schraubverschluss aufweisen.

Darüber hinaus kann die schlauch- oder rohrförmige Struktur mit einem Sammelgefäß oder -beutel integriert sein, insbesondere im Bereich von dessen Zulaufanschluss.

Andererseits ist es auch möglich, dass die schlauch- oder rohrförmige Struktur einen von ihrer Ablaufseite her durch die Abzweigung mit der Adsorptions- und/oder Absorptions- und/oder Filtereinrichtung kommunizierende Schlauch oder Rohrfortsatz aufweist.

Ein solcher, mit der Adsorptions- und/oder Absorptions- und/oder Filtereinrichtung kommunizierender Schlauch oder Rohrfortsatz kann in ein Sammelgefäß oder in einen Sammelbeutel hineinragen, insbesondere bis jenseits einer dortigen rückschlaghemmenden Folie oder einer anderen rücklaufhemmenden Struktur.

Außerdem kann die schlauch- oder rohrförmige Struktur mit einer im Rektum eines Patienten verankerten oder verankerbaren Kopfeinheit integriert oder verbindbar sein, welche über ein im Rektum platziertes oder platzierbares Ballonelement im Rektum verankerbar ist.

Die Geruchsbildung kann bei der dauerhaften Anwendung eines stuhlableitenden Systems ferner von dem stuhlableitenden, zwischen dem Patienten und dem Beutel angeordneten Schlauch ausgehen, wobei insbesondere die Barriereeigenschaft des verwendeten Schlauchmaterials eine entscheidende Rolle spielt. Herkömmliche Systemen, die in der Regel aus silikonbasierten Materialien bestehen, sind ohne zusätzliche Beschichtung, wie beispielsweise mit Parylene-basierten Stoffen, für eine geruchsdichte, kontinuierliche Stuhlableitung ungeeignet. Die schlechten Barriereeigenschaften von Silikon sind durch die ausgeprägte Porosität der verwendeten Silikone bedingt. Auch aufwendige Beschichtungen der Silokonoberfläche bieten in der Regel keinen ausreichenden Geruchsbarriere-Effekt. In jüngster Zeit werden neuartige silikon-basierte Systeme vorgestellt, die über einen speziellen, separat hergestellten und als separate Komponente verbauten Innenschlauch aus nylon- oder auch teflonartigem Material verfügen und so eine deutlich verbesserte Geruchsbarriere ermöglichen.

Zur Verbesserung der Geruchsdichtung können alternativ zu silikon-basierten, Schlauchkomponenten auch besonders kostengünstige, PVC-basierte oder mit einer PVC-Lage ko-extrudierte Schlauchmaterialien verwendet werden. Deren Barriereeffekt ist auch ohne zusätzliche Beschichtungs- oder Innenschlauchtechnik für die Mehrzahl der Stühle ausreichend effizient.

Die beschriebene Vorrichtung zur Dekompression von Darmgas und zur Adsorption von Geruchsstoffen kommt daher bevorzugt im Verbund mit PVC-basierten, PVC-haltigen oder mit PVC-Lagen koextrudierten, stuhlableitenden Schlauchleitungen zum Einsatz.

Um das Diffundieren von Geruchsstoffen durch zu- oder ableitende Schläuche, eine damit integrierte oder verbindbare Schlauch- oder Kopfeinheit und/oder einen damit integrierten oder verbindbaren Sammelbeutel zu minimieren, kann die schlauch- oder rohrförmige Struktur und/oder ein damit integriertes oder verbindbares Sammelgefäß und/oder ein damit integrierter oder verbindbarer Sammelbeutel und/oder eine damit integrierte oder verbindbare Schlauch- oder Kopfeinheit aus einem PVC-basierten Material oder aus einem PVC-haltigen oder mit einer oder mehreren PVC-Lagen beschichteten oder koextrudierten Material bestehen.

Die erfindungsgemäße Vorrichtung kann baulich seriell oder parallel zum stuhlableitenden System angeordnet sein.

Im Rahmen der Erfindung ist weiterhin vorgesehen, dass der abgeleitete Stuhl oder die abgeleiteten Exkrete oder Sekrete derart durch die schlauch- oder rohrförmige sowie T-förmige Struktur der Vorrichtung geführt sind, dass bei annähernd vertikaler Normallage der Vorrichtung eine Verschmutzung oder ein Verschluss der gasablassenden Komponenten der Vorrichtung weitgehend vermieden sind, wobei insbesondere Stauungen von beispielsweise Darmgas innerhalb des Systems vermieden werden können. Die Erfindung geht dabei davon aus, dass bei der Drainage der Stuhl oder die sonstigen Exkrete oder Sekrete unter dem Einfluss der Schwerkraft duch die Drainageleitung zu dem Sammelbehälter geleitet werden. Daher ist für den Betrieb des Systems eine vertikale Ausrichtung der Anordnung bevorzugt, und also auch eine vertikale Ausrichtung des Sammelbehälters. Für eben diese vertikale Ausrichtung sind Vorkehrungen getroffen, um eine Verschmutzung oder einen Verschluss der gasablassenden Komponenten der Vorrichtung weitgehend zu vermeiden.

Um eben eine solche vertikale Ausrichtung des Sammelbeutels sicherzustellen, sieht die Erfindung vor, dass an der schlauch- oder rohrförmigen sowie T-förmigen Struktur ein Haken zur vertikal hängenden Befestigung des Sammelbeutels vorgesehen ist.

Die Erfindung lässt sich dahingehend weiterbilden, dass der Haken derart weit auskragend ausgebildet ist, dass bei einem flach abgelegten Sammelbeutel dadurch dennoch die an dem Sammelbeutel angeschlossene schlauch- oder rohrförmige sowie T-förmige Struktur in stromaufwärtiger Richtung ansteigend geneigt ist, so dass trotz einer nicht vollkommen vertikalen Ausrichtung der erfindungsgemäße Geruchsverschluss funktioniert.

Schließlich entspricht es der Lehre der Erfindung, dass der Haken als doppelseitiger, in diametrale Richtungen auskragender Haken oder als um die Längsachse der schlauch- oder rohrförmigen sowie T-förmigen Struktur rotierbarer sowie ggf. in vorgegebenen Positionen einrastbarer Haltewinkel ausgebildet ist. Somit ist es für die Funktionsweise unerheblich, auf welche Seite der Sammelbeutel gelegt wird; stets erfährt die an den Sammelbeutel angeschlossene, schlauch- oder rohr- oder T-förmige Struktur eine Ausrichtung mit einer ausreichend vertikalen Komponente, um die Funktionsweise des Geruchsverschlusses sicherzustellen. Im Fall eines doppelseitigen, über beide Flachseiten des Sammelbeutels auskragenden Hakens wird dabei dem Anwender jede Sorgfalt über eine ausreichend vertikale Anordnung abgenommen; bei einem drehverstellbaren Haken muss der Anwender Sorge dafür tragen, dass der Haken zu der jeweils unten liegenden Flachseite des Sammelbeutels hin geschwenkt ist.

Weitere, bauliche und funktionelle Merkmale der erfindungsgemäßen, geruchsadsorbierenden, und/oder filternden, und/oder gasableitenden Einheit ergeben sich aus der folgenden Beschreibung bevorzugter Ausführungsformen der Erfindung sowie anhand der Zeichung. Hierbei zeigt:
- Fig. 1a: eine laterale, seitliche Anordnung der entgasenden Einheit zum stuhlableitenden Grundelement der Vorrichtung;
- Fig. 1b: eine Ausführungsform der erfindungsgemäßen Vorrichtung nach Art eines T-Stücks, wobei die dekomprimierende und/oder filternde Einheit reversibel lösbar oder auch fix auf dem freien Ende des seitlich abgehenden Schenkels aufgesteckt bzw. auf der Außenwandung des Schenkels konnektiert ist;
- Fig. 1c: eine weitere Ausführungsform der erfindungsgemäßen Vorrichtung nach Art eines T-Stücks, wobei die jeweilige dekomprimierende und/oder filternde Substanz in fixer Bauweise in das innere Lumen des lateralen Schenkels des T-Stücks integriert ist;
- Fig. 2: eine in das stuhlableitende Lumen des Systems einsteckbare Vorrichtung zur Dekompression, Adsorption und/oder Filterung von Darmgas;
- Fig. 3a: einen bespielhaften Aufbau einer lateral zum stuhlableitenden Lumen angeordneten, entgasenden Einheit, mit schichtartig angelegter, mäandernd gerichteter Gasführung durch das Adsorber- und/oder Filtermaterial;
- Fig. 3b: eine besondere Kombination einer fix in das Lumen des Grundelements integrierten Entgasungseinheit mit einer akzessorischen, von Beutel zu Beutel wechselbaren Einheit mit vergrößerter Adsorber bzw. Filterleistung;
- Fig. 3c: eine Bauweise der erfindungsgemäßen Vorrichtung mit spiraliger Gasführung durch das adsorbierende bzw. filternde Medium;
- Fig. 4: eine erfindungsgemäße Vorrichtung, welche eine schlauch- oder rohrartige, oder beispielsweise auch U-förmig profilierte, gasableitende Komponente als Verbindung zwischen dem stuhlaufnehmenden Raum eines Sammelbeutels und der dem Beutel vorgeschalteten, gasablassenden Einheit herstellt;
- Fig. 5: eine weitere Ausführungsform der erfindungsgemäßen Vorrichtung, bei der das gasableitende T-Stück in fixer Bauweise in die Einlass-Portion eines sammelnden Gefäßes eingebaut, und die Adsorber- und/oder Filterfunktion in einer Verschlusskappe integriert ist, welche während der Sammlungsphase auf dem lateralen Schenkel des T-Stücks aufsitzt und mit Abschluss der Sammlung auf den patientenseitigen, nach oben gerichteten Schenkel des T-Stücks für einen sicheren Verschluss des Beutels aufgesetzt wird;
- Fig. 6a: ein akzessorisches Adsorber- und/oder Filtermodul, welches über eine in das Gehäuse des Moduls integrierte Reservoir- und Dispenser-Komponente duftende bzw. geruchsneutralisierende Stoffe freisetzt;
- Fig. 6b: ein Dispenser-Modul, welches der gasablassenden Öffnung der Vorrichtung aufgesetzt bzw. in direkter Verlängerung nachgeschaltet wird, und über eine integrierte Reservoir-Funktion geruchsneutralisierende Duftstoffe freisetzt;
- Fig. 7: ein Ausführungsform einer ventilartig, flussrichtend wirkenden Doppellage von Folien; sowie
- Fig. 8: eine Ausführungsform eines beutelartigen Sammelgefäßes.

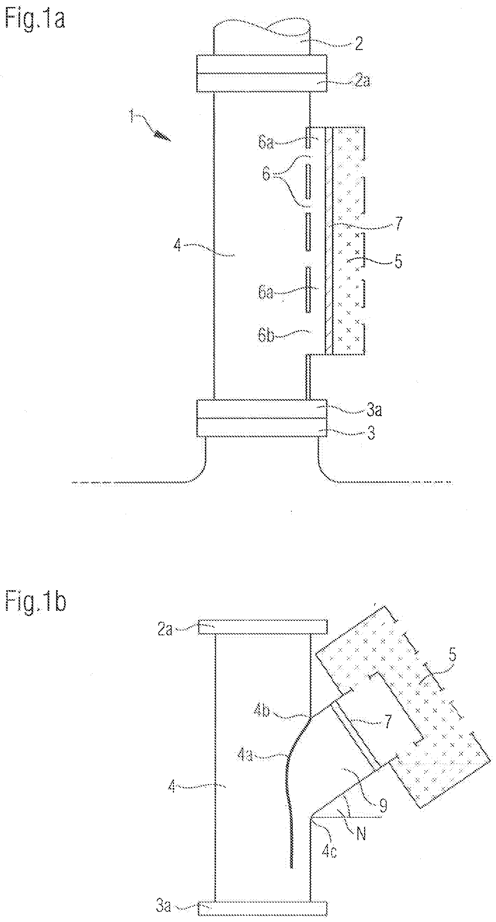

Fig. 1a zeigt eine stuhlableitende, Darmgas zur Umgebung ableitende Vorrichtung 1, die als separate, modulare Einheit zwischen dem beutelseitigen Ende 2 der Drainageleitung 12 und dem stuhlaufnehmenden Einlass 3 des Sammelbeutels 13 angeordnet ist. Die Vorrichtung 1 verfügt jeweils endständig, zur Drainageleitung 12 und zum Sammelbeutel 13 gerichtet, über konnektierende Komponenten 2a und 3a, mit denen die Vorrichtung 1 in serieller Anordnung, bevorzugt im Bereich des Übergangs von der Drainage zum Sammelbeutel 13 bzw. zum stuhlaufnehmenden Behälter, in das stuhldrainierende System eingefügt werden kann. Ein Grundelement 4 verbindet die konnektierenden Komponenten 2a und 3a derart, dass diese in einer miteinander kommunizierenden Verbindung stehen.

An den äußeren Umfang der Wandung des rohrartig zylindrischen Grundelementes 4 der Vorrichtung ist ein entgasendes, adsorbierendes und/oder filterndes Modul 5 in reversibel lösbarer, oder auch in fest mit dem Grundelement 4 verbundener Form angebracht. Das Modul 5 schmiegt sich der Wandung des Grundelementes 4 zirkulär geschlossen als Manschette, oder auch als nicht-geschlossene, C-förmige Schale an. Das Grundelement 4 verfügt im Bereich der Anbindung des Filtermoduls 5 über eine oder mehrere Öffnungen 6, denen sich ein spaltförmiger Zwischenraum 6a anschließt, der zum adsorbierenden bzw. filternden Material weisend, durch eine wasserundurchlässige, aber gasdurchlässige Trennlage 7 begrenzt ist. Die Trennlage 7 kann z.B. aus einem GoreTex-artigen Material bestehen, welches auf der dem Zwischenraum 6a zugewandten Seite durch einen schmutzabweisenden, beispielsweise lotusartigen Effekt ergänzt sein kann. Alternativ kann ein kostengünstiges, HME-Filter-typisches Vliespapier als Trennlage 7 verwendet werden, das ebenfalls wasserabweisend und zugleich luftdurchlässig wirkt. Konzeptionell ist die dem Stuhl zugewandte Seite der Trennlage 7 bevorzugt derart ausgeführt, dass eine Exposition mit Stuhl nicht zum dauerhaften, gasdichten Verschluss der Trennlage 7 führt. Vorzugsweise am unteren, zum Beutel 13 hin gerichteten Ende des Zwischenraumes 6a geht dieser über eine Öffnung 6b in den stuhlführenden bzw. stuhlableitenden Raum des Grundelementes 4 über. Stuhl, der über die Öffnungen 6 in den Zwischenraum 6a gelangt, kann somit über die Öffnung 6b zurück in das Lumen des Grundelementes 4 abfließen. In Abwandlung dieser Bauform kann das stuhlführende Lumen des Grundelementes 4 auch lediglich über die Öffnung 6b mit dem Zwischenraum 6a verbunden sein, also keine Öffnungen 6 aufweisen. Der Zwischenraum 6a erstreckt sich dann von der Öffnung 6b kaminartig nach oben, wodurch die oberhalb der Öffnung 6b liegende Trennlage 7 in senkrechter Normallage der Vorrichtung 1 vor einer direkten Stuhlexposition geschützt ist.

Fig. 1b zeigt eine Ausführungsform mit einem T-Stück-artigen Grundelement 4 mit einem lateralen, sich zur Umgebung hin öffnenden Schenkel 9, an welchem das adsorbierende und/oder filternde Modul 5 aufgebracht ist. Der Schenkel 9 ist in der bevorzugten Ausführung dieser Bauform in einem Neigungswinkel N zur Senkrechten bzw. zur Längsachse des Grundelementes 4 nach oben bzw. in Drainagerichtung nach stromaufwärts gerichtet, von ca. 15 bis 60 Grad, bevorzugt 30 bis 45 Grad. Das Modul 5 ist somit bei einer hängenden, annähernd senkrechten Normallage der Vorrichtung 1 bzw. der Einheit von stuhl-ableitendem Drainageschlauch 12, entgasender Vorrichtung 1 und sammelndem Beutel 13, vor dem direkten Einstrom von Stuhl geschützt. Stuhl, der bereits in den Schenkel 9 eingedrungen ist, kann von dort, der Neigung des Schenkels 9 folgend, in das Lumen des Grundelementes 4 ablaufen. Ergänzend zur beschriebenen Neigung des entgasenden Schenkels, kann der Übergang zum stuhlführenden Lumen des Grundelementes 4 durch eine Zunge 4a in Form einer vorhang- oder baldachinartigen Struktur geschützt sein, um den Eintritt von Stuhl in den entgasenden Schenkel 9 noch effizienter zu vermeiden. Die Zunge 4a als abschirmende Struktur geht beispielsweise in dem Übergangsbereich 4b zwischen dem nach lateral weisenden Schenkel 9 und dem senkrecht nach oben bzw. nach stromaufwärts verlaufenden Schenkel des T-förmigen Grundelements 4 aus der Innenwandung des Grundelementes 4 hervor, und reicht über den unteren Übergang 4c des nach lateral weisenden Schenkels 9 herab, bis in den senkrecht nach unten bzw. in Drainagerichtung nach stromabwärts verlaufenden Schenkel des T-förmigen Grundelements 4 hinein.

Alternativ zur Positionierung einer modularen Vorrichtung 5 zwischen Drainageleitung 12 und Sammelbeutel 13 als eine im Bedarfsfall seriell zuschaltbare Funktionseinheit, kann das Grundelement 4 auch direkt in den oberen Einlassbereich 3 des Sammelgefäßes 13 in fixer Bauart integriert sein. Im Falle eines beutelartigen Containers 13 wird dessen Folienkörper durch eine direkte Verschweißung oder Klebung mit dem nach unten gerichteten, zum Beutel 13 weisenden Schenkel des T-Stücks dicht schließend verbunden.

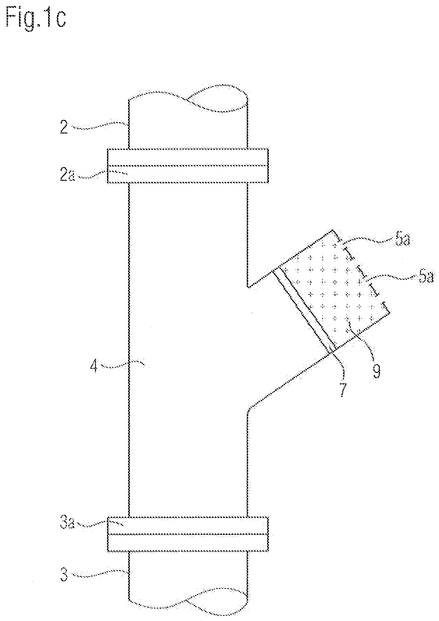

Fig. 1c zeigt eine an Fig.1b angelehnte Ausführungsform der Vorrichtung 1, bei der das adsorbierende und/oder filternde Material in fixer Bauweise bereits in den lateralen, das Gesamtsystem von Drainage 12 und Beutel 13 zur Umgebung hin öffnenden, gasableitenden Schenkel 9 des T-Stück-förmigen Grundelements 4 integriert ist. Der Schenkel ist dabei derart bemessen, dass er z.B. ca. 5 bis 10 ml Aktivkohle-Granulat aufnimmt. Das Kohlegranulat wird zum stuhlführenden, oberen und unteren Schenkel des T-Stück-förmigen Grundelements 4 durch eine gasdurchlässige, wasserabweisende Trennlage 7 abgegrenzt. Die stirnseitige Öffnung des Schenkels 9 ist mit einem kappenartigen Verschluss versehen, der über Öffnungen 5a für den Gasauslass zur Umgebung verfügt.

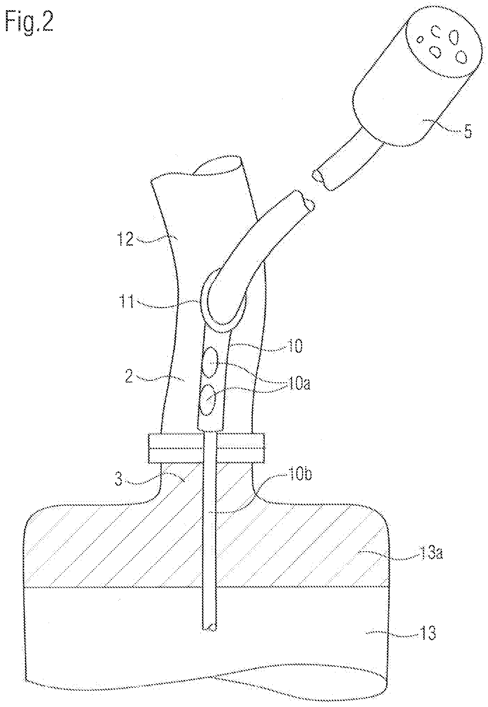

Fig. 2 zeigt eine Ausführung der Vorrichtung 1, wobei der Auslass von Darmgas durch einen schlauchartigen oder rohrartigen Einschub 10 hergestellt wird, der in die stuhlableitende Einheit eingesteckt wird. Der Einschub 10 wird über eine passgenau gestaltete Öffnung 11 eingeführt, welche im unbenutzten Zustand beispielsweise mit einem kappenartigen Element verschlossen wird. Das distale Ende des Einschubs 10 wird in der bevorzugten Ausführungsform bis in den stuhlaufnehmenden Binnenraum des Beutels 13 vorgeschoben, wobei er eventuelle rückschlaghemmende Folienlagen 13a überragt, so dass die Folien die retrograd gerichtete Ableitung von Gas aus dem Beutel 13 nicht behindern und somit bereits in den Beutel gelangtes Gas zur Umgebung hin abgeleitet werden kann. Das proximale Ende des Einschubs 10 weist endständig ein fix angebrachtes Adsorber- und/oder Filtermodul 5 auf. Der Einschub erfolgt optional im beutelnahen Bereich 2 des stuhlableitenden Katheter-Schlauches 12, bevorzugt aber im Bereich der Koennektion des Drainagekatheters 12 zum Sammelbeutel 13, beispielsweise durch eine in den jeweiligen Konnektor bzw. Anteile des Konnektors integrierte Öffnung. Der Einschub 10 verfügt in der bevorzugten Ausführung über möglichst großlumige Öffnungen 10a, die oberhalb der rückschlaghemmenden Folien 13a in den stuhlführenden Raum münden. Der schlauch- oder rohrartige Anteil des Einschubs 10b, der über die rückschlaghemmenden Folien hinaus in den sammelnden Raum des Beutels 13 reicht, ist bevorzugt mit einem kleineren Lumen versehen, um einen eventuellen Rückstrom von flüssigem Inhalt zu begrenzen. Das Modul 5 wird ca. 10 cm oberhalb der Öffnung 11 positioniert, um bei senkrechter Normallage des Beutels 13 eine Verschmutzung durch den Beutelinhalt zu vermeiden.

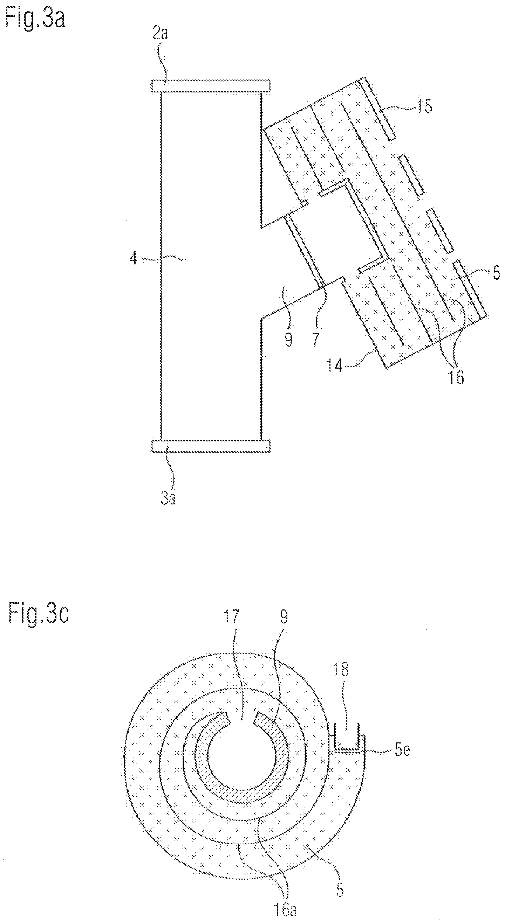

Fig. 3a zeigt eine Fig. 1b entsprechende Ausführungsform der gasablassenden Vorrichtung 1 mit einem T-Stück-artigen Grundelement 4, wobei dem lateralen Schenkel 9 ein Modul 5 mit Adsorber- und/oder Filtermaterial dicht schließend aufsitzt. Das Modul 5 weist im Inneren lamellenartige Strukturen 16 auf, die das aus dem System strömende Gas möglichst effizient, bei bestmöglicher Nutzung der geruchsbindenden und/oder filternden Kapazität, vom Modulboden 14 zum Moduldeckel 15 durch den Modulinhalt führen. Die Lamellen 16 können beispielsweise horizontal versetzt, fingerartig ineinandergreifen oder auch spiralförmig oder wendelartig geführt sein. Im bevorzugten Fall ist das Modul 5 derart zum Grundelement 4 angeordnet, dass die durch das Modul 5 auf die Einheit von Drainagekatheter 12, entgasender Vorrichtung 1 und sammelndem Beutel 13 wirkende Hebelkraft möglichst klein gehalten wird, ein Abkippen der in der Regel hängend befestigten Einheit nach frontal also vermieden wird. Das Modul 5 sollte eine entsprechend flache, dem Grundelement 4 möglichst angeschmiegte, den Schwerpunkt möglichst nicht versetzende Bauweise haben. Der Inhalt an Aktivkohle im Modul 5 der beschriebenen Bauweise sollte bei 20 bis 100 ml, bevorzugt bei 30 bis 50 ml liegen.

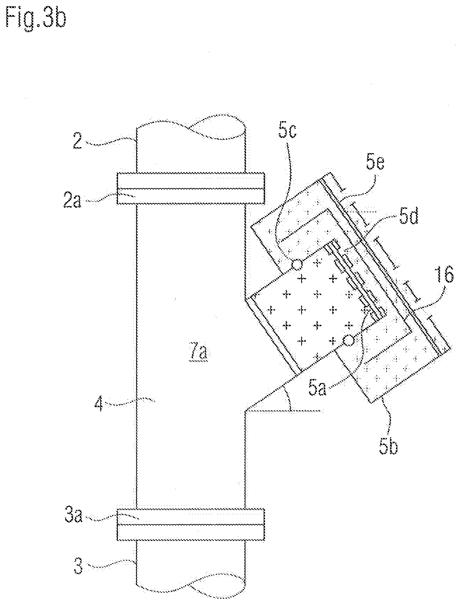

Fig. 3b zeigt eine Kombination eines fix in den lateralen Schenkel 9 verbauten Adsorbers bzw. Filters entsprechend Fig. 1a, wobei auf den Schenkel 9 eine akzessorische, filternde und/oder adsorbierende Filterkartusche 5b als zusätzliche Einheit in reversibel lösbarer Weise aufgesteckt werden kann. Die Kartusche 5b ist zur Optimierung der Dichtung im Fügebereich zwischen Schenkel 9 und Kartusche 5b mit einer dichten Lippe oder einer O-Ring-artigen Dichtung 5c versehen. Das strinseitig aus dem Schenkel 9 austretende Gas wird über entsprechende Öffnungen 5d in der buchsenartigen Vertiefung des Bodens der Kartusche 5b aufgenommen, und durch lamellenartige Stege 16, im Sinne einer möglichst effizienten Adsorption durch die adsorbierende bzw. filternde Masse hindurch geleitet. Um den Austritt von Keimen oder Viren aus dem System in die Umgebung zu verhindern, ist vor den Austrittsöffnungen des Kartuschendeckels entsprechend sporen-, bakterien- und virendichtes Filtermaterial 5e vorgelagert.

Fig. 3c zeigt eine besondere Form der Gasführung durch das Filter- bzw. Adsorbermaterial, wobei das Innere des Moduls 5 mit einer spiral- oder schneckenförmig trennenden Wandung 16a versehen ist, die sich vom Zentrum bzw. von der Einlassöffnung nach außen hin erweitert und sich über eine weitere Öffnung 17 endständig zur Umgebung hin öffnet. Zur Vermeidung des Austritts infektiöser Erreger wie Viren oder bakterielle Sporen ist der Öffnung 17 ein entsprechend filterndes Material 5e vorgelagert.

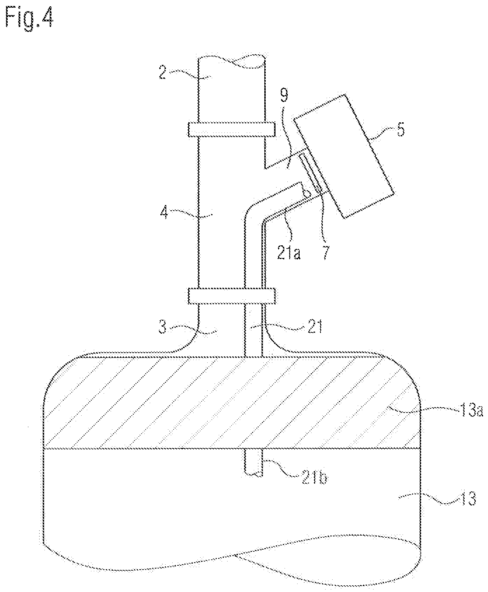

Fig. 4 zeigt eine schlauch- oder rohrartige, flexibel oder starr ausgeführte Komponente 21, deren zum Patienten gewandtes Ende 21a fest an der Innenwandung des Grundelementes 4, bevorzugt im Bereich des lateralen Schenkels 9 angebracht ist, und deren proximales Ende 21b über das untere Ende des Grundelementes 4 hinaus, in den stuhlsammelnden Raum des Sammelgefäßes 13 eintaucht. Die Komponente 21 kann dabei rückschlaghemmende Komponenten, wie beispielsweise lappenartige, einander dicht schließend anliegende und ventilartig wirkende Folienlagen 13a überwinden, so dass in den Beutel 13 eingedrungenes Darmgas durch das Lumen der Komponente 21 hindurch, und/oder an der Außenseite des Komponente 21 entlang, vorzugsweise direkt in den gasablassenden Schenkel 9 der Vorrichtung 1 entleert werden kann.

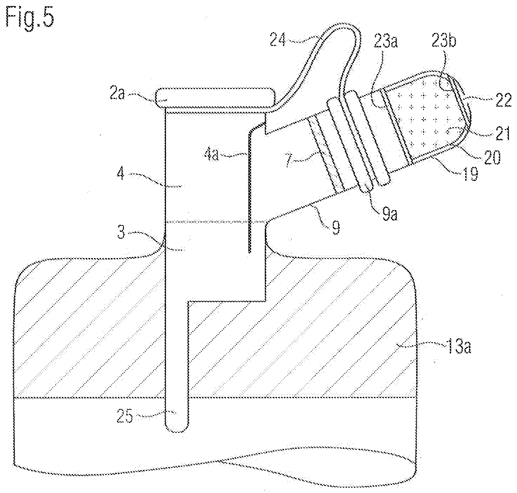

Fig. 5 zeigt eine weitere Bauform einer erfindungsgemäßen Vorrichtung 1, wobei das T-Stück-artige Grundelement 4 fest in die Einlass-Portion 3 des Beutels 13 integriert, vorzugsweise eingeschweißt oder eingeklebt ist. Der laterale Schenkel 9 ist mit einem bajonettartigen Verschlussmechanismus 9a versehen, der dem Verschlussmechanismus 2a zum Anschluss des Drainage-Katheters 12 an den Beutel 13 entspricht. Während der Phase der Stuhl-Sammlung wird der laterale Schenkel 9 mit einer Verschlusskappe 19 verschlossen. Die Kappe 19 integriert eine Kartusche 20 mit Filtermaterial 21 auf Basis einer Aktivkohle. Den vorausgehenden Ausführungen entsprechend, ist die freie Öffnung des Schenkels 9 mit einer gasdurchlässigen, aber wasser- und/oder schmutzabweisenden Trennlage 7 versehen, die das Austreten von Stuhl aus dem T-Stück-artigen Grundelement 4 vermeidet. Die Verschlusskappe 19 wird gasdicht schließend mit dem Schenkel 9 konnektiert. Die Kappe 19 weist endständig eine Öffnung 22 für den Gasauslass auf. Alternativ zu einem kartuschenartigen Gefäß, kann der Binnenraum der Kappe 19 direkt mit Kohle bzw. Adsorber- oder Filtermaterial befüllt sein, wobei der befüllte Raum zu beiden Seiten 23a und 23b beispielsweise mit stabilen, aber gasdurchlässigen Trennlagen aus porösem Sintermaterial verschlossen sein kann. Im Falle der Verwendung einer Aktivkohle beträgt das verbaute Volumen 4 bis 10 ml, bevorzugt 5 bis 6 ml.

Wird der Beutel 13 von der Drainage 12 getrennt und entsorgt, wird die Verschlusskappe 19 vom Schenkel 9 gelöst, auf den Anschluss 2a aufgesetzt und dort dicht schließend konnektiert. Die Trennlage 7 verhindert dann den freien Ausstrom von Flüssigkeit durch den seitlichen Schenkel 9.

Um das Eindringen von abgeleitetem Stuhl in den lateralen Schenkel 9 zu erschweren, ist im inneren des T-Stück-artigen Grundelements 4 ferner eine den Übergang vom stuhlführenden Weg zum lateral abzweigenden Schenkel 9 schützende Zunge 4a in Form einer vorhangartigen oder siphonartigen Struktur angebracht.

Um Darmgas abzuleiten, das bereits in den sammelnden Raum des Beutels 13 vorgedrungen ist, bzw. um Gas über eine im sammelnden Beutel 13 integrierte, rückschlaghemmende Funktion, wie zum Beispiel rückschlaghemmende Folienlagen 13a in das der Rückschlaghemmung vorgeschaltete, T-Stück-förmige Grundelement 4 zurückzuleiten, und von dort aus durch den Adsorber und/oder Filter 5 zur Umgebung hin abzuführen, verfügt das T-Stück-förmige Grundelement 4 an seinem proximalen Ende über einen stabartigen, rohr- oder schlauchartigen Fortsatz 25, der über eine rückschlaghemmende Folie oder sonstige Funktion hinweg in den sammelnden Beutel 13 reicht. Das Profil des Fortsatzes 25 ist derart gestaltet, dass Gas entweder um den Fortsatz 25 herum oder durch den Fortsatz 25 hindurch vom sammelnden Binnenraum des Beutels 13 in dem Beutel 13 vorgeschaltete Bereiche geleitet werden kann. In der beschriebenen Bauform mit einem fix in die Einlass-Portion 3 des Beutels 13 integrierten T-Stück 4 kann daher auf einen, in konventionellen Sammelbeuteln 13 üblichen, direkt die Beutelwandung integrierten Auslass auf vorteilhafte Weise verzichtet werden. Auslasskomponenten, die in innerhalb des sammelnden Kompartiments in dessen Wandung integriert sind, sind insbesondere dann problematisch, wenn der Sammelbeutel 13 in eine flach liegende Position gerät und Beutelinhalt den Filter dann gegebenenfalls benetzt oder durchtränkt wodurch der Filter seine Entgasungsfunktion partiell oder vollständig verliert.

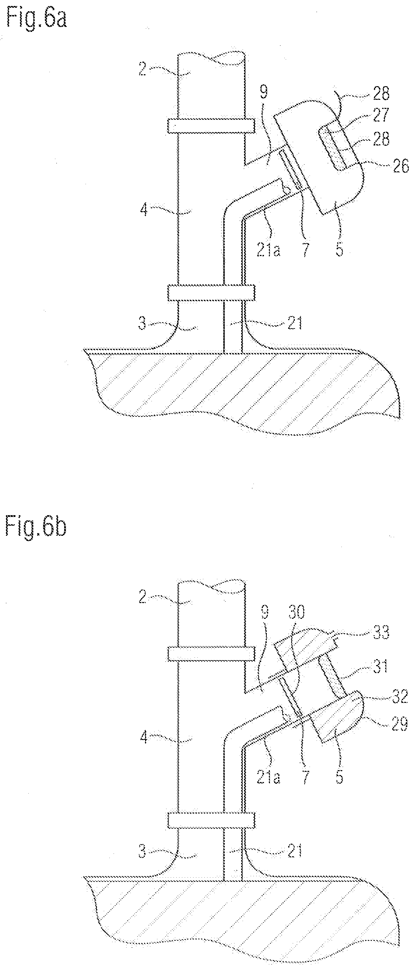

Abb. 6a zeigt eine abgewandelte Bauform eines Moduls 5, 5b, welches neben einer adsorbierenden und/oder filternden Wirkung eine einen Duftstoff freisetzende Funktion aufweist. Die Freisetzung von parfümierend wirkenden bzw. störende Gerüche übertönend wirkenden Stoffen erfolgt aus einem Kompartiment 26, welches in das Gehäuse des Moduls 5 integriert, von dessen adsorbierendem bzw. filterndem Medium jedoch durchgängig separiert ist. Im Kompartiment 26 ist eine saugfähige Speicherkomponente 27 aus beispielsweise mehrlagig gepresster Zellulose eingelegt, die den Duftstoff über ihre entsprechend große Gesamtoberfläche aufnimmt und über ihre zur Umgebung weisende Vorderfläche verzögert freisetzt. Das Kompartiment 26 kann mit einer Abziehfolie 28 versiegelt sein. Die Freisetzung eines Duftstoffes kann auch aus einem separaten, beispielsweise kapselartigen Behälter 28 erfolgen, der in eine entsprechende muldenartige Aussparung des Moduls 5 eingesetzt wird. Der jeweilige Duftstoff kann vom Anwender aus einer Mehrzahl von Duftvarianten ausgewählt und gewechselt werden.

Fig. 6b beschreibt ein akzessorisches Dispenser-Modul 29, welches der gasablassenden Öffnung 30 der Vorrichtung 1 bzw. des T-Stück-artigen Grundelements 4 direkt aufgesetzt bzw. jener unmittelbar nachgeschaltet angeordnet wird, und über eine integrierte substanzfreisetzende Funktion kontinuierlich geruchsneutralisierende Duftstoffe in den unmittelbaren Auslassbereich der Vorrichtung 1 freisetzt. Darmgas, welches durch die gasablassende Öffnung 30 aus der Vorrichtung 1 bzw. dem stuhldrainierenden Gesamtsystem austritt, passiert in dieser besonderen Ausführungsform ein vlies-artiges, großporiges oder weitmaschiges Gewebe 31, das den Duftstoff in ähnlicher Weise wie ein Docht aus einer Reservoir-Komponente 32 aufnimmt und über die entsprechend große Oberfläche der porigen oder auch netzartigen Struktur des Vlies in das austretende Gas abgibt und so dessen geruchswirksame Inhaltsstoffe durch entsprechend wirksame Wohlgerüche übertönt.

Der freigesetzte Duftstoff basiert bevorzugt auf flüchtigen, ölbasierten Lösungen mit ausreichend guten Laufeigenschaften im freisetzenden Vlies. Das mit dem vliesartigen Gewebe 31 verbundene Reservoir 32 besteht beispielsweise aus einer filzartigen oder schwammartigen Komponente, und kann optional über eine kleinlumige Öffnung 33 von außen - bspw. durch einen ampullenartigen, zwischen den Fingern ausdrückbaren Behälter - unmittelbar vor der Kombination des Moduls 5 mit der Drainage-Vorrichtung 1 bzw. dem T-stück-artigen Grundelement 4 mit Duftstoff betankt werden. Der Anwender kann bei diesem System zwischen mehreren Duftvarianten wählen. Er kann das Modul 5 ferner beim Wechsel des Sammelbeutels 13 oder des Gesamtsystems vom Beutel 13 oder vom System abnehmen und auf einen neuen Beutel 13 oder ein neues System übertragen. Das Modul 5 kann also bei einem Patienten über die Gesamtdauer der Anwendung der Drainage-Vorrichtung 1 verwendet werden. Ist das Reservoir erschöpft, kann es durch die Öffnung erneut mit Duftstoff befüllt werden.

Somit beschreibt die Erfindung eine akzessorisch anwendbare oder fest in ein Stuhl oder sonstige Sekrete oder Exkrete ableitendes System integrierte Vorrichtung zur Bindung und/oder Filterung von Gasen, welche sich beispielsweise im Rahmen einer kontinuierlichen, geschlossenen Stuhlableitung innerhalb eines stuhldrainierenden Systems sammeln, und wobei diese Gase ohne Freisetzung störender Gerüche zur Umgebung hin ableitet bzw. dekomprimiert werden, wobei die Vorrichtung baulich seriell oder parallel zum stuhlableitenden System angeordnet ist, wobei die Ableitung des Gases zur Umgebung hin außerhalb des sammelnden Raumes des Sammelgefäßes 13 erfolgt, und die jeweilige gasablassende Komponente oder Funktion nicht mit gesammeltem Inhalt verschmutzt und somit funktionsunfähig werden kann.

Bevorzugt erfolgt die Ableitung von Gas zur Umgebung unmittelbar oberhalb des sammelnden Raumes, beispielsweise im stuhlableitendenden Schlauch des Systems zu dem sammelnden Raum.

Bereits in den sammelnden Raum des Gefäßes 13 eingedrungenes Gas kann, beispielsweise durch einen stabartigen, rohr- oder schlauchartigen Fortsatz, in den Bereich des Gasauslasses abgeleitet und von dort dekomprimiert werden.

Die gasablassende Funktion des filternden und/oder absorbierenden Materials wird durch eine schmutz- und oder wasserabweisende Trennlage zwischen dem stuhlableitenden Binnenraum der Vorrichtung und dem jeweils konnektierten Filter- bzw. Absorbermodul 5 aufrechterhalten.

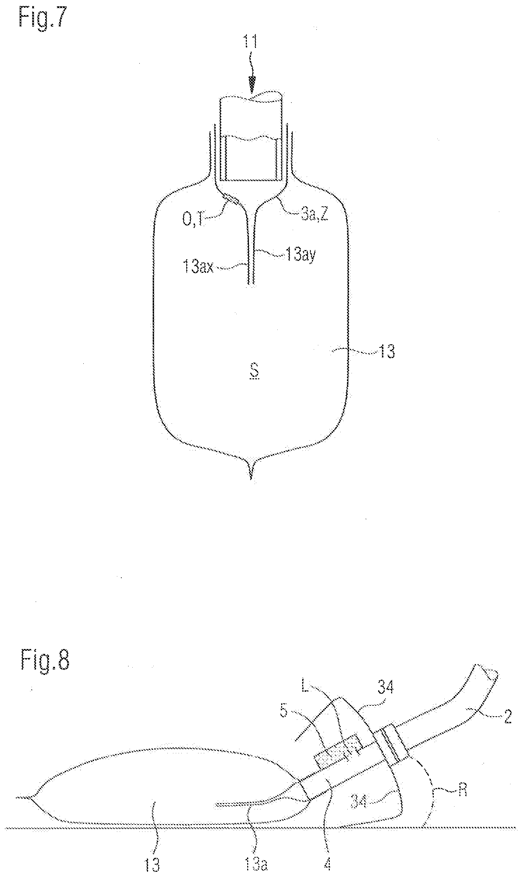

Fig. 7 zeigt eine besondere Ausführung einer ventilartig, flussrichtend wirkenden Doppellage von Folien, die einen Rückstrom von Gas aus dem Sammelbehälter 13 in das zum Beutel zuleitende System erlaubt.

Fig. 8 zeigt eine winkelartige Haltevorrichtung zur Fixierung des Beutels in vertikal hängender Position, wobei der Rückstrom von Beutelinhalt in das zum Beutel zuleitende System bei passagerer flacher Lage des Beutels erschwert wird.

Fig. 7 zeigt eine ventilartig, flussrichtend wirkende Anordnung von zwei, einander flächig anliegenden Folienlagen 13ax und 13ay, die im inneren des Sammelbehälters 13 in verbaut sind. Zum Sammelgefäß ablaufendes Material M tritt durch die beiden Folienlagen hindurch in den sammelnden Raum S des Behälters ein. Füllt sich das Gefäß in der vertikal hängenden oder stehenden Normalposition bis auf Höhe der beiden Folienlagen bzw. über diese hinaus, verursacht der flüssige Inhalt einen lippenartigen Schluss der Folienalgen, und der ungewünschte Ausstrom bzw. Rückstrom von Inhalt aus dem Sammelbehälter durch die zuleitende Öffnung wird verhindert. Ein entsprechender Effekt stellt sich ein, wenn gasförmige Substanzen in das Sammelgefäß gelangen. Die Gasfüllung führt ebenfalls zum dichten Verschluss der beiden Folienlagen, wodurch sich das Gas sukzessive im Gefäß staut und der schließlich resultierende Verschlussdruck auf die beiden Folienlagen derart ausgeprägt ist, dass ein weiterer Einstrom von flüssigem Inhalt in das sammelnde Gefäß nicht mehr möglich ist. Ohne entsprechende bauliche Modifikation der Anti-Rückschlag Funktion, muss das Sammelgefäß in diesem Falle gewechselt werden. Um Gas, welches bereits in den Beutel vorgedrungen ist, aus dem Beutel abzuleiten, sieht die Erfindung speziell ausgeführte Folienlagen vor, die zwar flüssigkeitsdicht sind, jedoch Gase frei passieren lassen. Die Folienlagen können flächig, z.B. vollständig aus einem entsprechenden Material bestehen, oder die entsprechenden Eigenschaften auch nur anteilig in bestimmten Segmenten aufweisen. Vorteilhaft wird die gasdurchlässige Funktion oder Einheit dort platziert, wo ein vollständiger Verschluss der beiden Folienlagen auch bei maximaler Füllung des Gefäßes mechanisch bedingt ausgeschlossen ist. Dies ist im Bereich der Anbindung bzw. des Übergangs 3a der Folienlagen zu einem rohr- oder stutzenartigen Einlauf- oder Konnektorsegment gegeben, wo die dichtenden Folienlagen beispielsweise angeschweißt oder angeklebt sind. Bei einem Außendurchmesser des zuleitenden Elementes von ca. 15 bis 25 mm stellt sich i.d.R. ein zwickelförmiger, offener, auch bei maximaler Füllung des Gefäßes nicht schließender Bereich Z ein, der eine Höhe von mindestens 5 bis 15 mm aufweist, und somit ausreichend Fläche für die beschriebene Funktion bzw. Integration einer "gasdurchlässigen Trennlage" T bietet. Alternativ zu gasdurchlässigem Folienmaterial, kann im zwickelförmigen Bereich Z auch eine kleine, freie Öffnung O, von beispielsweise 1 bis 2 mm Durchmesser positioniert sein. Im bevorzugten Falle wird jedoch im beschriebenen zwickelförmigen Bereich eine einfache oder kreuzförmige Schlitzung mit einer scharfen Klinge durchgeführt, wobei die Schenkel der Schlitzung jeweils etwa 5 mm Länge aufweisen. Die Schlitzung kann zwar den Rückstrom von Inhalt in das Segment oberhalb des sammelnden Raumes nicht vollständig verhindern, schließt aber einen höher-volumigen Rückstrom von bereits gesammeltem Inhalt aus.

Abb. 8 zeigt eine Ausführungsform eines beutelartigen Sammelgefäßes 13, wobei in horizontaler Lage des Beutels 13 der Ausstrom von Beutelinhalt in das zuleitende Segment 4 des Beutels 13 durch eine rampenartige Steigung R des zuleitenden Segmentes 4 erschwert, reduziert bzw. verhindert wird. Hierzu wird der winkelförmige Haken 34 zur vertikal hängenden Befestigung des Beutels 13 derart ausgeführt, dass er das zuleitende bzw. auch gasablassende Segment 4, relativ zum flach liegenden Beutel 13, in eine schräge, aufsteigend gerichtete Position bringt. Das entstehende Gefälle gewährleistet, dass flüssiger Beutelinhalt, über eine gewisse Phase der Flachlagerung des Beutels 13 hinweg, die gasablassende Öffnung L im zuleitenden Segment 4 des Sammelbeutels 13 möglichst nicht erreicht. Der winkelförmige Haken 34 ist bevorzugt doppelseitig, zu beiden Flachseiten eines beispielsweise aus zwei Lagen verschweißten Beutels 13 hin ausgeführt, so dass sich die erwünschte Schräge in jedem Falle der Flachlagerung des Beutels 13 einstellt. Der Haken 34 bzw. Doppelhaken ist in der bevorzugten Bauweise in einem fixen 90 Grad Winkel zu den Beutelflächen angebracht. Die für die Schräge erforderliche 90 Grad Position kann auch durch einen axial rotierbaren, in 90 Grad Position schwenkbaren und dort einrastenden Haltewinkel erreicht werden.

**Bezugszeichenliste**

| | | | |
|---|---|---|---|
| 1 | Vorrichtung | 12 | Drainageleitung |
| 2 | Ende | 13 | Beutel, Sammelgefäß |
| 2a | Konnektor | 13a | Folienlage |
| 3 | Einlass | 13a | Folienlage |
| 3a | Konnektor | 13a | Folienlage |
| 4 | Grundelement | 14 | Modulboden |
| 4a | Zunge | 15 | Moduldeckel |
| 4b | Übergangsbereich | 16 | Lamelle |
| 4c | Übergang | 16a | Wandung |
| 5 | Modul | 17 | Öffnung |
| 5a | Öffnung | 19 | Verschlusskappe |
| 5b | Filterkartusche | 20 | Kartusche |
| 5c | Dichtung | 21 | Komponente |
| 5d | Öffnung | 21a | Ende |
| 5e | Filtermaterial | 21b | Ende |
| 6 | Öffnung | 22 | Öffnung |
| 6a | Zwischenraum | 23a | Seite |
| 6b | Öffnung | 23b | Seite |
| 7 | Trennlage | 25 | Fortsatz |
| 9 | Schenkel | 26 | Kompartiment |
| 9a | Verschlussmechanismus | 27 | Speicherkomponente |
| 10 | Einschub | 28 | Abziehfolie, Kapsel |
| 10a | Öffnung | 29 | Dispenser-Modul |
| 10b | Einschub | 30 | Öffnung |
| 11 | Öffnung | 31 | Gewebe |
| 32 | Reservoir | | |
| 33 | Öffnung | | |
| 34 | Haken | | |
| L | Öffnung | | |
| M | Material | | |
| N | Neigungswinkel | | |
| O | Öffnung | | |
| R | Steigung | | |
| S | Raum | | |
| T | Trennlage | | |
| Z | Bereich | | |

## Patentansprüche

1. System zur geschlossenen Ableitung und/oder Sammlung von Stuhl oder sonstigen, stark geruchsbildenden Exkreten oder Sekreten und zur möglichst geruchsneutralen Ableitung von Darmgas oder sonstigen, übelriechende Gasen durch eine natürliche Öffnung des Körpers eines menschlichen oder tierischen Patienten, mit einem ableitenden, schlauch- oder rohrförmigen Drainagekatheter (12), einer stromabwärts des Drainagekatheters (12) positionierten Vorrichtung (1) zur möglichst geruchsneutralen Ableitung des Darmgases oder der sonstigen, übelriechenden Gase, und einem stromabwärts der Vorrichtung (1) und daran extrakorporal angeschlossenen Sammelbeutel (13), um den Stuhl oder die abgeleiteten Exkrete oder Sekrete in ein Sammelkompartiment innerhalb des Sammelbeutels (13) abzuleiten, wobei der Sammelbeutel (13) über eine rückflusshemmende Funktion verfügt, die bereits in den Sammelbeutel (13) gelangtes Material dort hält, auch wenn der Sammelbeutel (13) aus der vertikalen Normallage in eine flach liegende Position gelangt, wobei die Vorrichtung (1) eine schlauch- oder rohrförmige Struktur (4) nach Art eines T-Stücks aufweist, welche neben (i) einem distalen, zum Patienten gerichteten Ende als Zulaufanschluss, (ii) einem proximalen, dem Zulaufanschluss in axialer Verlängerung gegenüber stehenden, zum Sammelbeutel (13) gerichteten Ende als Ablaufanschluss und (iii) einem jene verbindenden Durchleitungsbereich für die Durchleitung von Stuhl oder sonstigen Exkreten oder Sekreten eine lateral zur Umgebung gerichtete Abzweigung (9) aufweist, wo Darmgas oder sonstige, übelriechende Gase von dem Stuhl oder den sonstigen Exkreten oder Sekreten getrennt und durch eine Adsorptions- und/oder Absorptions- und/oder Filtereinrichtung (5) zu einem Auslass geleitet werden, und wobei gewährleistet ist, dass in das Sammelkompartiment innerhalb des Sammelbeutels (13) vorgedrungenes Gas die rückflusshemmende Funktion überwindet und der dem Sammelbeutel (13) vorgeschalteten gasablassenden Abzweigung (9) zugeführt wird.

2. System nach Anspruch 1, **dadurch gekennzeichnet, dass** das rückschlaghemmende Element folienbasierte, lippen- oder segelartige Komponenten (13ax,13ay) aufweist, vorzugsweise wobei sich der Abstand zwischen den folienbasierten, lippen- oder segelartigen Komponenten (13ax,13ay) in der Hauptströmungsrichtung, also in Richtung von der Drainageleitung (12) zu dem Sammelkompartiment des Sammelbehälters (13), verjüngt.

3. System nach einem der vorhergehenden Ansprüche, **dadurch gekennzeichnet, dass** die Adsorptions- und/oder Absorptions- und/oder Filtereinrichtung (5) zumindest bereichsweise von dem Durchleitungsbereich beabstandet ist, um einen intensiven Kontakt mit den Durchleitungsbereich durchströmendem Stuhl oder sonstigen Exkreten oder Sekreten so weit als möglich zu vermeiden.

4. System nach einem der vorhergehenden Ansprüche, **dadurch gekennzeichnet, dass** die Längsrichtung des Strömungskanals zwischen der Abzweigung (9) und der Adsorptions- und/oder Absorptions- und/oder Filtereinrichtung mit der Längsrichtung des Durchleitungsbereichs zwischen der Abzweigung (9) und dem Ablaufanschluss einen Winkel von 90° oder weniger als 90° einschließt, oder einen Winkel von 75° oder weniger, oder einen Winkel von 60° oder weniger, oder einen Winkel von 45° oder weniger, oder sogar einen Winkel von 30° oder weniger.

5. System nach einem der vorhergehenden Ansprüche, **dadurch gekennzeichnet, dass** der maximale Strömungsquerschnitt in dem Bereich zwischen der Abzweigung (9) und der Adsorptions- und/oder Absorptions-und/oder Filtereinrichtung (5) gleich oder kleiner ist als der minimale Strömungsquerschnitt in dem Durchleitungsbereich.

6. System nach einem der vorhergehenden Ansprüche, **dadurch gekennzeichnet, dass** der Strömungsquerschnitt in dem Bereich zwischen der Abzweigung (9) und der Adsorptions- und/oder Absorptions- und/oder Filtereinrichtung (5) gegenüber dem minimalen Strömungsquerschnitt in dem Durchleitungsbereich durch ein Hindernis verengt ist, vorzugsweise wobei das Hindernis als eine Zunge (4a) ausgebildet ist, die
a) innerhalb des Durchleitungsbereichs etwa auf Höhe der Abzweigung (9) angeordnet ist, und/oder
b) stromaufwärts der Abzweigung (9) angelenkt ist und die Abzweigung (9) zu der Adsorptions- und/oder Absorptions- und/oder Filtereinrichtung (5) gegenüber dem Durchleitungsbereich im Falle der Durchleitung von Stuhl oder sonstigen Exkreten oder Sekreten zumindest bereichsweise abdeckt.

7. System nach Anspruch 6 mit einem Hindernis in Form einer Zunge (4a), **dadurch gekennzeichnet, dass** die Zunge (4a) biegsam und/oder elastisch ausgebildet ist, und/oder vorzugsweise aus Kunststoff besteht, vorzugsweise wobei die Zunge (4a)
a) mit der schlauch- oder rohrförmigen, nach Art eines T-Stücks gestalteten Struktur (4) integriert oder durch Klebung oder eine andere Fügetechnik verbunden ist, und/oder
b) über eine steife Verbindung an der schlauch- oder rohrförmigen, nach Art eines T-Stücks gestalteten Struktur (4) festgelegt ist, und/oder
c) ein oder mehrere Löcher (6) zum Durchtritt von Gasen aufweist.

8. System nach einem der vorhergehenden Ansprüche, **dadurch gekennzeichnet, dass** die dem Durchleitungsbereich oder der Abzweigung (9) zugewandte Einlassöffnung der Adsorptions- und/oder Absorptions- und/oder Filtereinrichtung (5) durch eine gasdurchlässige, vorzugsweise aber wasserabweisende Trennlage (7) abgedeckt ist, vorzugsweise wobei die Trennlage (7)
a) eine gering benetzbare Oberfläche aufweist wie bei einem Lotoseffekt, insbesondere an ihrer dem Durchleitungsbereich oder der Abzweigung (9) zugewandten Seite, und/oder
b) als mikroporöse Membran ausgebildet ist, beispielsweise aus expandiertem Polytetrafluorethylen, bevorzugt mit einem maximalen Porendurchmesser von weniger als 1 mm, oder mit einem maximalen Porendurchmesser von 100 µm oder weniger, oder mit einem maximalen Porendurchmesser von 10 µm oder weniger, und/oder
c) aus einem Papier gebildet ist, insbesondere aus einem Vliespapier.

9. System nach einem der vorhergehenden Ansprüche, **dadurch gekennzeichnet, dass** die Adsorptions- und/oder Absorptions- und/oder Filtereinrichtung (5) eine oder mehrere oberflächenaktive, die Geruchsstoffe auf der Oberfläche anlagernde, adsorbierenden Substanzen, aufweist, und/oder eine oder mehrere absorbierende, die geruchswirksamen Stoffe in Art einer physikalischer Lösung aufnehmende Substanzen.

10. System nach einem der vorhergehenden Ansprüche, **dadurch gekennzeichnet, dass** die Adsorptions- und/oder Absorptions- und/oder Filtereinrichtung (5) ein adsorbierendes oder absorbierendes oder filterndes Granulat umfasst, beispielsweise Aktivkohle, beispielsweise in einer Menge von 100 ml oder weniger, oder in einer Menge von 50 ml oder weniger, oder in einer Menge von 20 ml oder weniger, oder in einer Menge von 10 ml oder weniger, oder gar in einer Menge von 5 ml oder weniger, vorzugsweise wobei die Adsorptions- und/oder Absorptions- und/oder Filtereinrichtung (5)
a) das Granulat, beispielsweise die Aktivkohle, in einer Menge von 2 ml oder mehr enthält, oder in einer Menge von 5 ml oder mehr, oder in einer Menge von 10 ml oder mehr, oder in einer Menge von 20 ml oder mehr, oder gar in einer Menge von 30 ml oder mehr, und/oder
b) in ihrem Inneren ein oder mehrere lamellenartige Strukturen (16) aufweist, um das zu reinigende Gas durch die Adsorptions- und/oder Absorptions- und/oder Filtereinrichtung (5) zu führen, insbesondere wobei mehrere lamellenartigen Strukturen (16) gegeneinander versetzt sind und/oder nach Art von verschränkten Fingern ineinander greifen, und/oder
c) in ihrem Inneren wenigstens eine schnecken-, spiral- und/oder wendelförmige Struktur (16a) aufweist, um das zu reinigende Gas durch die Adsorptions- und/oder Absorptions- und/oder Filtereinrichtung (5) zu führen, und/oder
d) ein infektiöse Erreger filterndes Material aufweist, insbesondere im Bereich ihrer stromabwärtigen bzw. auslassseitigen Öffnung (5a,5d).

11. System nach einem der vorhergehenden Ansprüche, **dadurch gekennzeichnet, dass** die Adsorptions- und/oder Absorptions- und/oder Filtereinrichtung (5) in einer auswechselbaren Kartusche (5b,20) oder in einer abnehmbaren Kappe (19) angeordnet ist, vorzugsweise wobei die auswechselbare Kartusche (5b,20) oder die abnehmbaren Kappe (19)
a) im Bereich ihrer freien Stirnseite oder ihres Umfangs mit einer oder mehreren Auslassöffnungen versehen ist, und/oder
b) über einen Bajonettverschluss oder über einen Schraubverschluss mit der schlauch- oder rohrförmigen, nach Art eines T-Stücks gestalteten Struktur (4) an deren Abzweigung (9) verbindbar ist, und/oder
c) über ihren Verschluss wahlweise auch mit dem Zulaufanschluss (2a) der schlauch- oder rohrförmigen, nach Art eines T-Stücks gestalteten Struktur (4) verbindbar ist.

12. System nach einem der vorhergehenden Ansprüche, **dadurch gekennzeichnet, dass** die schlauch- oder rohrförmige, nach Art eines T-Stücks gestaltete Struktur (4) und/oder die auswechselbare Kartusche (5b.20) und/oder die abnehmbaren Kappe (19) wenigstens ein Kompartiment (26) zur Aufnahme eines Duftstoffs aufweist, vorzugsweise wobei das Kompartiment (26) zur Aufnahme eines Duftstoffs
a) nach außen offen ist und/oder mit einer abnehmbaren Abdeckung, beispielsweise in Form einer Abziehfolie (28), verschlossen ist, und/oder
b) ringförmig ausgebildet ist und eine zentrale Auslassöffnung (30) der Adsorptions- und/oder Absorptions- und/oder Filtereinrichtung (5) umgibt, insbesondere wobei innerhalb der zentralen Öffnung (30) des ringförmigen Kompartiments (26) zur Aufnahme eines Duftstoffs ein Docht oder ein vliesartiges und/oder weitmaschiges und/oder großporiges Gewebe (31) angeordnet ist, bspw. ein quer über die zentrale Öffnung (30) gespannter Docht oder ein vliesartiges und/oder weitmaschiges und/oder großporiges Gewebe (31), der (das) sich zu seiner Befeuchtung mit Duftstoff bis in das den Duftstoff enthaltende Kompartiment (26) hinein erstreckt.

13. System nach einem der vorhergehenden Ansprüche, **dadurch gekennzeichnet, dass** die schlauch- oder rohrförmige, nach Art eines T-Stücks gestaltete Struktur (4) an ihrem Zulaufanschluss (2a) und/oder an ihrem Ablaufanschluss (3a) einen Bajonettverschluss oder einen Schraubverschluss zum Anschluss eines Schlauchs (12) oder Sammelgefäßes oder -beutels (13) od. dgl. aufweist, vorzugsweise wobei die schlauch- oder rohrförmige, nach Art eines T-Stücks gestaltete Struktur (4)
a) mit einem Sammelgefäß oder -beutel (13) integriert ist, insbesondere im Bereich von dessen Zulaufanschluss (3), und/oder
b) einen von ihrer Ablaufseite her durch die Abzweigung (9) mit der Adsorptions- und/oder Absorptions- und/oder Filtereinrichtung (5) kommunizierende Schlauch oder Rohrfortsatz (21) aufweist, insbesondere wobei der mit der Adsorptions- und/oder Absorptions-und/oder Filtereinrichtung (5) kommunizierende Schlauch oder Rohrfortsatz (21) in ein Sammelgefäß oder in einen Sammelbeutel (13) hineinragt, insbesondere bis jenseits einer dortigen rückschlaghemmenden Folie (13a,13ax,13ay) oder einer anderen rücklaufhemmenden Struktur, und/oder
c) mit einer im Rektum eines Patienten verankerten oder verankerbaren Kopfeinheit integriert oder verbindbar ist, welche über ein im Rektum platziertes oder platzierbares Ballonelement im Rektum verankerbar ist.

14. System nach einem der vorhergehenden Ansprüche, **dadurch gekennzeichnet, dass** die schlauch- oder rohrförmige Struktur und/oder ein damit integriertes oder verbindbares Sammelgefäß und/oder ein damit integrierter oder verbindbarer Sammelbeutel (13) und/oder eine damit integrierte oder verbindbare Schlauch- oder Kopfeinheit aus einem PVC-basierten Material oder aus einem PVC-haltigen oder mit einer oder mehreren PVC-Lagen beschichteten oder koextrudierten Material besteht.

15. System nach einem der vorhergehenden Ansprüche, **dadurch gekennzeichnet, dass** sie baulich seriell oder parallel zum stuhlableitenden System angeordnet ist.

16. System nach einem der vorhergehenden Ansprüche, **dadurch gekennzeichnet, dass** der abgeleitete Stuhl oder die abgeleiteten Exkrete oder Sekrete derart durch die schlauch- oder rohr- oder T-Stück-förmige Struktur (4) der Vorrichtung geführt sind, dass bei annähernd vertikaler Normallage der Vorrichtung (1) eine Verschmutzung oder ein Verschluss der Adsorptions-und/oder Absorptions- und/oder Filtereinrichtung (5) der Vorrichtung (1) weitgehend vermieden sind, wobei insbesondere Stauungen von beispielsweise Darmgas innerhalb des Systems vermieden werden können.

17. System nach einem der vorhergehenden Ansprüche, **dadurch gekennzeichnet, dass** an der schlauch- oder rohr- oder T-Stück-förmigen Struktur (4) ein Haken (34) zur vertikal hängenden Befestigung des Beutels (13) vorgesehen ist, vorzugsweise wobei der Haken (34)
a) derart weit auskragend ausgebildet ist, dass bei einem flach abgelegten Sammelbeutel (13) dadurch die an dem Sammelbeutel (13) angeschlossene schlauch- oder rohr- oder T-Stück-förmige Struktur (4) in stromaufwärtiger Richtung ansteigend geneigt ist, und/oder
b) als doppelseitiger, in diametrale Richtungen auskragender Haken oder als um die Längsachse der schlauch- oder rohrförmigen, nach Art eines T-Stücks gestalteten Struktur (4) rotierbarer sowie ggf. in vorgegebenen Positionen einrastbarer Haltewinkel ausgebildet ist.

## Claims

1. System for the closed removal and/or collection of faeces or other strongly odour-forming excretions or secretions and for removing intestinal gas or other malodorous gases in as odour-neutral a manner as possible through a natural opening of the body of a human or animal patient, having a removing, hose- or tube-like drainage catheter (12), an apparatus (1), positioned downstream of the drainage catheter (12), for removing the intestinal gas or the other malodorous gases in as odour-neutral a manner as possible, and a collection bag (13) arranged downstream of the apparatus (1) and connected thereto outside the body for removing the faeces or the removed excretions or secretions into a collection compartment within the collection bag (13), wherein the collection bag (13) has a backflow-inhibiting function which retains material that has already entered the collection bag (13) in the collection bag even if the collection bag (13) moves from the vertical normal position into a position in which it lies flat, wherein the apparatus (1) has a hose- or tube-like structure (4) in the manner of a T-piece, said structure having, in addition to (I) a distal end, oriented towards the patient, as an inflow port, (II) a proximal end, located opposite the inflow port as an axial prolongation thereof and oriented towards the collection bag (13), as an outflow port, and (III) a passage region, connecting said ends, for the passage of faeces or other excretions or secretions, a branch (9), oriented laterally to the surroundings, where intestinal gas or other malodorous gases are separated from the faeces or the other excretions or secretions and are guided through an adsorption and/or absorption and/or filter device (5) to an outlet, and wherein it is ensured that gas that has reached the collection compartment within the collection bag (13) overcomes the backflow-inhibiting function and is conveyed to the gas-discharging branch (9) upstream of the collection bag (13).

2. System according to claim 1, **characterised in that** the return-inhibiting element has foil-based, lip- or sail-like components (13ax, 13ay), preferably wherein the distance between the foil-based, lip- or sail-like components (13ax, 13ay) becomes narrower in the main direction of flow, that is to say in the direction from the drainage line (12) to the collection compartment of the collection container (13).

3. System according to any one of the preceding claims, **characterised in that** the adsorption and/or absorption and/or filter device (5) is spaced apart from the passage region at least in some regions in order to avoid as far as possible intensive contact with faeces or other excretions or secretions flowing through the passage region.

4. System according to any one of the preceding claims, **characterised in that** the longitudinal direction of the flow channel between the branch (9) and the adsorption and/or absorption and/or filter device encloses an angle of 90° or less than 90°, or an angle of 75° or less, or an angle of 60° or less, or an angle of 45° or less, or even an angle of 30° or less, with the longitudinal direction of the passage region between the branch (9) and the outflow port.

5. System according to any one of the preceding claims, **characterised in that** the maximum flow cross section in the region between the branch (9) and the adsorption and/or absorption and/or filter device (5) is equal to or less than the minimum flow cross section in the passage region.

6. System according to any one of the preceding claims, **characterised in that** the flow cross section in the region between the branch (9) and the adsorption and/or absorption and/or filter device (5) is narrowed relative to the minimum flow cross section in the passage region by a barrier, preferably wherein the barrier is in the form of a tongue (4a), which
a) is arranged within the passage region approximately at the height of the branch (9), and/or
b) is articulated upstream of the branch (9) and at least in some regions covers the branch (9) to the adsorption and/or absorption and/or filter device (5) with respect to the passage region in the case of the passage of faeces or other excretions or secretions.

7. System according to claim 6 having a barrier in the form of a tongue (4a), **characterised in that** the tongue (4a) is flexible and/or elastic, and/or consists preferably of plastics material, preferably wherein the tongue (4a)
a) is integrated with the hose- or tube-like structure (4) configured in the manner of a T-piece or is connected thereto by adhesive bonding or another joining technique, and/or
b) is fixed to the hose- or tube-like structure (4) configured in the manner of a T-piece via a rigid connection, and/or
c) has one or more holes (6) for the passage of gases.

8. System according to any one of the preceding claims, **characterised in that** the inlet opening, facing the passage region or the branch (9), of the adsorption and/or absorption and/or filter device (5) is covered by a gas-permeable but preferably water-repellent separating layer (7), preferably wherein the separating layer (7)
a) has a surface with low wettability, as in the case of a lotus effect, in particular on its side facing the passage region or the branch (9), and/or
b) is in the form of a microporous membrane, for example of expanded polytetrafluoroethylene, preferably with a maximum pore diameter of less than 1 mm, or with a maximum pore diameter of 100 pm or less, or with a maximum pore diameter of 10 pm or less, and/or
c) is formed of a paper, in particular of a nonwoven paper.

9. System according to any one of the preceding claims, **characterised in that** the adsorption and/or absorption and/or filter device (5) has one or more surface-active adsorbent substances that take up the odorous substances on the surface, and/or one or more absorbent substances that absorb the odour-active substances in the manner of a physical solution.

10. System according to any one of the preceding claims, **characterised in that** the adsorption and/or absorption and/or filter device (5) comprises an adsorbent or absorbent or filtering granulate, for example activated carbon, for example in an amount of 100 ml or less, or in an amount of 50 ml or less, or in an amount of 20 ml or less, or in an amount of 10 ml or less, or even in an amount of 5 ml or less, preferably wherein the adsorption and/or absorption and/or filter device (5)
a) contains the granulate, for example the activated carbon, in an amount of 2 ml or more, or in an amount of 5 ml or more, or in an amount of 10 ml or more, or in an amount of 20 ml or more, or even in an amount of 30 ml or more, and/or
b) has in its interior one or more lamellar structures (16) for guiding the gas to be cleaned through the adsorption and/or absorption and/or filter device (5), in particular wherein a plurality of lamellar structures (16) are offset relative to one another and/or mesh with one another in the manner of interlaced fingers, and/or
c) has in its interior at least one screw-like, spiral and/or helical structure (16a) for guiding the gas to be cleaned through the adsorption and/or absorption and/or filter device (5), and/or
d) has a material that filters infectious pathogens, in particular in the region of its downstream or outlet-side opening (5a, 5d).

11. System according to any one of the preceding claims, **characterised in that** the adsorption and/or absorption and/or filter device (5) is arranged in a replaceable cartridge (5b, 20) or in a removable cap (19), preferably wherein the replaceable cartridge (5b, 20) or the removable cap (19)
a) is provided in the region of its free end face or of its circumference with one or more outlet openings, and/or
b) can be connected by way of a bayonet closure or by way of a screw closure to the hose- or tube-like structure (4) configured in the manner of a T-piece at the branch (9) thereof, and/or
c) can be connected via its closure optionally also to the inflow port (2a) of the hose- or tube-like structure (4) configured in the manner of a T-piece.

12. System according to any one of the preceding claims, **characterised in that** the hose- or tube-like structure (4) configured in the manner of a T-piece and/or the replaceable cartridge (5b, 20) and/or the removable cap (19) has at least one compartment (26) for accommodating an aromatic substance, preferably wherein the compartment (26) for accommodating an aromatic substance
a) is open to the outside and/or is closed by a removable cover, for example in the form of a tear-off film (28), and/or
b) is of annular form and surrounds a central outlet opening (30) of the adsorption and/or absorption and/or filter device (5), in particular wherein there is arranged inside the central opening (30) of the annular compartment (26) for accommodating an aromatic substance a wick or a nonwoven-like and/or widemeshed and/or large-pored fabric (31), for example a wick that is stretched transversely across the central opening (30) or a nonwoven-like and/or widemeshed and/or large-pored fabric (31), said wick or fabric extending into the compartment (26) containing the aromatic substance so as to be wetted with aromatic substance.

13. System according to any one of the preceding claims, **characterised in that** the hose- or tube-like structure (4) configured in the manner of a T-piece has at its inflow port (2a) and/or at its outflow port (3a) a bayonet closure or a screw closure for connection of a hose (12) or collection vessel or bag (13) or the like, preferably wherein the hose- or tube-like structure (4) configured in the manner of a T-piece
a) is integrated with a collection vessel or bag (13), in particular in the region of the inflow port (3) thereof, and/or
b) has a hose or tube extension (21) communicating from the outflow side thereof, through the branch (9), with the adsorption and/or absorption and/or filter device (5), in particular wherein the hose or tube extension (21) communicating with the adsorption and/or absorption and/or filter device (5) projects into a collection vessel or into a collection bag (13), in particular beyond a backflow-inhibiting foil (13a, 13ax, 13ay) or other return-inhibiting structure provided there, and/or
c) is integrated with or can be connected to a head unit that is anchored or can be anchored in the rectum of a patient and that can be anchored in the rectum by way of a balloon element which is placed or can be placed in the rectum.

14. System according to any one of the preceding claims, **characterised in that** the hose- or tube-like structure and/or a collection vessel that is integrated therewith or can be connected thereto and/or a collection bag (13) that is integrated therewith or can be connected thereto and/or a hose unit or head unit that is integrated therewith or can be connected thereto consists of a PVC-based material or of a PVC-containing material or material coated or coextruded with one or more PVC layers.

15. System according to any one of the preceding claims, **characterised in that** it is arranged structurally in series or in parallel with the faeces-removing system.

16. System according to any one of the preceding claims, **characterised in that** the faeces that have been removed or the excretions or secretions that have been removed are guided through the hose- or tube- or T-piece-like structure (4) of the apparatus such that, when the apparatus (1) is in the approximately vertical normal position, contamination or closure of the adsorption and/or absorption and/or filter device (5) of the apparatus (1) is largely avoided, wherein in particular accumulations of, for example, intestinal gas within the system can be avoided.

17. System according to any one of the preceding claims, **characterised in that** there is provided on the hose- or tube- or T-piece-like structure (4) a hook (34) for fastening the bag (13) in a vertically suspended manner, preferably wherein the hook (34)
a) is configured to project sufficiently far that, when the collection bag (13) is laid flat, the hose- or tube- or T-piece-like structure (4) connected to the collection bag (13) is inclined upwards in the upstream direction, and/or
b) is in the form of a double-sided hook projecting in diametrically opposite directions or in the form of a mounting bracket that is rotatable about the longitudinal axis of the hose- or tube-like structure (4) configured in the manner of a T-piece and can optionally be engaged in predefined positions.

## Revendications

1. Système destiné à l'évacuation et/ou à la collecte en circuit fermé de selles ou d'autres excrétions ou sécrétions fortement odorantes, ainsi qu'à l'évacuation, dans la mesure du possible sans odeur, de gaz intestinaux ou d'autres gaz malodorants par un orifice naturel du corps d'un patient humain ou animal, comprenant un cathéter de drainage (12) en forme de tuyau ou de tube, un dispositif (1) positionné en aval du cathéter de drainage (12) pour l'évacuation, aussi inodore que possible, des gaz intestinaux ou d'autres gaz malodorants, et une proche de collecte (13) située en aval du dispositif (1) et raccordée à celui-ci de manière extracorporelle, afin de diriger les selles ou les excrétions et sécrétions vers un compartiment de collecte situé à l'intérieur de la poche de collecte (13), dans lequel la poche de collecte (13) est dotée d'un dispositif anti-reflux qui retient les matières déjà parvenues dans la poche de collecte (13), même si la poche de collecte (13) passe de sa position verticale normale à une position à plat, le dispositif (1) comportant une structure (4) en forme de tuyau ou tubulaire en forme de raccord en T, qui, outre (I) une extrémité distale tournée vers le patient servant de raccord d'amenée, (II) une extrémité proximale, située dans le prolongement axial du raccord d'amenée et orientée vers la poche de collecte (13) en tant que raccord de sortie, et (III) une zone de passage reliant celles-ci pour le passage des selles ou d'autres excrétions ou sécrétions, comporte une dérivation (9) orientée latéralement par rapport à l'environnement, dans laquelle les gaz intestinaux ou autres gaz malodorants sont séparés des selles ou d'autres excrétions ou sécrétions et acheminés vers une sortie par un dispositif d'adsorption et/ou d'absorption et/ou de filtration (5), et dans lequel ce qui garantit que le gaz qui a pénétré dans le compartiment de collecte à l'intérieur de la poche de collecte (13) surmonte la fonction anti-retour et est acheminé vers la dérivation d'évacuation des gaz (9) située en amont de la poche de collecte (13).

2. Système selon la revendication 1, **caractérisé en ce que** l'élément anti-retour comprend des composants (13ax, 13ay) constitués de feuille ne forme de voile ou de lèvre, de préférence, dans lequel la distance entre les éléments (13ax, 13ay) constitués de feuille en forme de lèvre ou de voile, diminue dans la direction principale de l'écoulement, notamment dans la direction allant de la conduite de drainage (12) vers le compartiment de collecte du récipient de collecte (13).

3. Système selon l'une quelconque des revendications précédentes, **caractérisé en ce que** le dispositif d'adsorption et/ou d'absorption et/ou de filtration (5) est placé à l'écart, au moins par endroits, de la zone de passage, pour empêcher autant que possible tout contact étroit avec les selles ou autres excrétions ou sécrétions transitant par la zone de passage.

4. Système selon l'une quelconque des revendications précédentes, **caractérisé en ce que** la direction longitudinale du canal d'écoulement entre la dérivation (9) et le dispositif d'adsorption et/ou d'absorption et/ou de filtration, avec la direction longitudinale de la zone de passage entre la dérivation (9) et le raccord de sortie, forme un angle de 90° ou inférieur à 90°, ou un angle inférieur ou égal à 75°, ou un angle inférieur ou égal à 60°, ou un angle inférieur ou égal à 45°, voire un angle inférieur ou égal à 30°.

5. Système selon l'une quelconque des revendications précédentes, **caractérisé en ce que** la section transversale maximale d'écoulement dans la zone située entre la dérivation (9) et le dispositif d'adsorption et/ou d'absorption et/ou de filtration (5) est inférieure ou égale à la section transversale minimale d'écoulement dans la zone de passage.

6. Système selon l'une quelconque des revendications précédentes, **caractérisé en ce que** la section d'écoulement dans la zone située entre la dérivation (9) et le dispositif d'adsorption et/ou d'absorption et/ou de filtration (5) est rétrécie par un obstacle par rapport à la section d'écoulement minimale dans la zone de passage, de préférence dans lequel l'obstacle est réalisé sous la forme d'une languette (4a) qui
a) est disposée à l'intérieur de la zone de passage, à peu près à la hauteur de la dérivation (9), et/ou
b) est articulée en amont de la dérivation (9) et recouvre, au moins à certains endroits, la dérivation (9) menant au dispositif d'adsorption et/ou d'absorption et/ou de filtration (5) par rapport à la zone de passage, en cas de passage de selles ou d'autres excrétions ou sécrétions.

7. Système selon la revendication 6, comprenant un obstacle sous la forme d'une languette (4a), **caractérisé en ce que** la languette (4a) est flexible et/ou élastique, et/ou est de préférence en matière plastique, de préférence dans lequel la languette (4a)
a) est intégrée à la structure (4) tubulaire ou en forme de tuyau, conçue à la manière d'un raccord en T, ou y est assemblée par collage ou par une autre technique d'assemblage, et/ou
b) est fixée par une liaison rigide à la structure tubulaire ou en forme de tuyau (4), conçue à la manière d'un raccord en T, et/ou
c) comporte un ou plusieurs orifices (6) destinés au passage des gaz.

8. Système selon l'une quelconque des revendications précédentes, **caractérisé en ce que** l'ouverture d'entrée du dispositif d'adsorption et/ou d'absorption et/ou de filtration (5), tournée vers la zone de passage ou la dérivation (9), est recouverte d'une couche de séparation (7) perméable aux gaz, mais de préférence hydrofuge, de préférence dans lequel la couche de séparation (7)
a) présente une surface peu mouillable, comme dans le cas d'un effet lotus, notamment sur sa face tournée vers la zone de passage ou la dérivation (9), et/ou
b) est constituée d'une membrane microporeuse, par exemple en polytétrafluoroéthylène expansé, de préférence dont le diamètre maximal des pores est inférieur à 1 mm, ou dont le diamètre maximal des pores est inférieur ou égal à 100 pm, ou dont le diamètre maximal des pores est inférieur ou égal à 10 pm, et/ou
c) est constituée d'un papier, en particulier d'un papier non tissé.

9. Système selon l'une des revendications précédentes, **caractérisé en ce que** le dispositif d'adsorption et/ou d'absorption et/ou de filtration (5) comprend une ou plusieurs substances tensioactives adsorbantes qui fixent les substances odorantes à leur surface, et/ou une ou plusieurs substances absorbantes qui capturent les substances odorantes sous forme d'une solution physique.

10. Système selon l'une quelconque des revendications précédentes, **caractérisé en ce que** le dispositif d'adsorption et/ou d'absorption et/ou de filtration (5) comprend des granulés adsorbants, absorbants ou filtrants,
par exemple du charbon actif, par exemple en une quantité de 100 ml ou moins, ou en une quantité de 50 ml ou moins, ou à raison de 20 ml ou moins, ou à raison de 10 ml ou moins, voire à raison de 5 ml ou moins, de préférence dans lequel le dispositif d'adsorption et/ou d'absorption et/ou de filtration (5)
a) contient les granulés, par exemple le charbon actif, à raison de de 2 ml ou plus, ou à raison de 5 ml ou plus, ou à raison de 10 ml ou plus, ou à raison de 20 ml ou plus, voire à raison de 30 ml ou plus, et/ou
b) comporte en son sein une ou plusieurs structures (16) en forme de lamelles afin de faire passer le gaz à purifier à travers le dispositif d'adsorption et/ou d'absorption et/ou de filtration (5), notamment dans lequel plusieurs structures (16) en forme de lamelles sont décalées les unes par rapport aux autres et/ou s'emboîtent les unes dans les autres à la manière de doigts entrelacés, et/ou
c) comporte en son sein au moins une structure (16a) hélicoïdale, en spirale et/ou en hélice afin de faire passer le gaz à purifier à travers le dispositif d'adsorption et/ou d'absorption et/ou de filtration (5), et/ou
d) comporte un matériau filtrant les agents infectieux, notamment au niveau de son ouverture aval ou côté sortie (5a, 5d).

11. Système selon l'une quelconque des revendications précédentes, **caractérisé en ce que** le dispositif d'adsorption et/ou d'absorption et/ou de filtration (5) est disposé dans une cartouche remplaçable (5b, 20) ou dans un capuchon amovible (19), de préférence dans lequel la cartouche remplaçable (5b, 20) ou le capuchon amovible (19)
a) est pourvu(e) d'un ou plusieurs orifices de sortie au niveau de sa face frontale libre ou de son pourtour, et/ou
b) peut être raccordé(e), au moyen d'un raccord à baïonnette ou d'un bouchon à vis, à la structure (4) tubulaire ou en forme de tuyau conçue à la manière d'un raccord en T, au niveau de sa dérivation (9), et/ou
c) peut également être raccordé(e), sélectivement, par l'intermédiaire de son bouchon, au raccord d'amenée (2a) de la structure (4) tubulaire ou en forme de tuyau, conçue à la manière d'un raccord en T.

12. Système selon l'une quelconque des revendications précédentes, **caractérisé en ce que** la structure (4) tubulaire ou en forme de tuyau, conçue à la manière d'un raccord en T et/ou la cartouche remplaçable (5b.20) et/ou le capuchon amovible (19) comporte au moins un compartiment (26) destiné à recevoir un parfum, de préférence dans lequel le compartiment (26) destiné à recevoir un parfum
a) est ouvert vers l'extérieur et/ou est fermé par un couvercle amovible, par exemple sous la forme d'un film pelable (28), et/ou
b) se présente sous forme annulaire et entoure une ouverture de sortie centrale (30) du dispositif d'adsorption et/ou d'absorption et/ou de filtration (5), en particulier dans lequel une mèche ou un tissu (31) de type non-tissé et/ou à mailles larges et/ou à larges pores est disposé(e) à l'intérieur de l'ouverture centrale (30) du compartiment annulaire (26) pour recevoir un parfum, par exemple, une mèche tendue en travers de l'ouverture centrale (30) ou un tissu (31) de type non-tissé et/ou à mailles larges et/ou à pores larges qui s'étend jusqu'au compartiment (26) contenant le parfum afin d'être imprégné(e) de celui-ci.

13. Système selon l'une quelconque des revendications précédentes, **caractérisé en ce que** la structure (4) tubulaire ou en forme de tuyau, conçue à la manière d'un raccord en T, comporte, au niveau de son raccord d'entrée (2a) et/ou de son raccord de sortie (3a), un bouchon à baïonnette ou un bouchon à vis pour le raccordement d'un tuyau (12) ou d'un récipient ou d'une poche de collecte (13) ou similaire, de préférence dans lequel la structure (4) tubulaire ou en forme de tuyau, conçue à la manière d'un raccord en T,
a) est intégrée à un récipient ou à une poche de collecte (13), notamment dans la zone de son raccord d'amenée (3), et/ou
b) comporte un prolongement de tuyau ou de tube (21) qui, du côté sortie, communique avec le dispositif d'adsorption et/ou d'absorption et/ou de filtration (5) par l'intermédiaire de la dérivation (9),
en particulier lorsque le tuyau ou le prolongement de tuyau (21) en communication avec le dispositif d'adsorption et/ou d'absorption et/ou de filtration (5) débouche dans un récipient de collecte ou dans une poche de collecte (13), notamment jusqu'à un film anti-retour (13a, 13ax, 13ay) ou une autre structure anti-retour qui s'y trouve, et/ou
c) qui peut être intégrée ou reliée à une unité principale ancrée ou pouvant être ancrée dans le rectum d'un patient, laquelle peut être ancrée dans le rectum au moyen d'un élément ballon placé ou pouvant être placé dans le rectum.

14. Système selon l'une quelconque des revendications précédentes, **caractérisé en ce que** la structure tubulaire ou en forme de tuyau et/ou un récipient de collecte intégré ou pouvant être raccordé à celle-ci et/ou une poche de collecte (13) intégrée ou pouvant être raccordée à celle-ci et/ou une unité de tuyau ou de tête intégrée ou pouvant être reliée à celle-ci est constituée d'un matériau à base de PVC ou d'un matériau contenant du PVC ou revêtu d'une ou plusieurs couches de PVC ou coextrudé.

15. Système selon l'une quelconque des revendications précédentes, **caractérisé en ce qu'**il est disposé en série ou en parallèle avec le système de dérivation des selles.

16. Système selon l'une quelconque des revendications précédentes, **caractérisé en ce que** les selles, les excrétions ou les sécrétions sont acheminées à travers la structure (4) en forme de tuyau, tubulaire ou de raccord en T (4) du dispositif de telle sorte que, lorsque le dispositif (1) se trouve dans une position normale approximativement verticale, une contamination ou une fermeture du dispositif d'adsorption et/ou d'absorption et/ou de filtration (5) du dispositif (1) soient largement évitées, ce qui permet notamment d'éviter des accumulations, par exemple de gaz intestinaux, à l'intérieur du système.

17. Système selon l'une quelconque des revendications précédentes, **caractérisé en ce qu'**un crochet (34) disposé sur la structure (4) en forme de tuyau, tubulaire tube ou de té permet de suspendre à la verticale la poche (13), de préférence dans lequel le crochet (34)
a) est conçu de manière à faire saillie suffisamment pour que, lorsque la poche de collecte (13) est posée à plat, la structure (4) en forme de tuyau ou tubulaire en forme de raccord en T reliée à la poche de collecte (13) soit inclinée vers le haut dans le sens amont, et/ou
b) est conçu sous la forme d'un crochet à double face en porte-à-faux dans des directions diamétrales, ou sous la forme d'une équerre de fixation pouvant pivoter autour de l'axe longitudinal de la structure (4) en forme de tuyau ou tubulaire en forme de raccord en **T,** et pouvant, le cas échéant, s'enclencher dans des positions prédéfinies.
